# EUROPEAN PATENT SPECIFICATION

(11) **EP 3 573 632 B1**
(45) Date of publication and mention of the grant of the patent: **07.08.2024**
(21) Application number: 18744813.9
(22) Date of filing: 30.01.2018
(51) Int. Cl.: C07K 14/535, C12N 15/86, A61K 35/76, A61P 35/00, C12N 7/00, A61K 35/761

(54) **TUMOR SELECTIVE TATA-BOX AND CAAT-BOX MUTANT ONCOLYTIC VIRUSES**
TUMORSELEKTIV ONKOLYTISCHES VIRUS MIT TATA-BOX- UND CAAT-BOX-MUTATIONEN
VIRUS ONCOLYTIQUES MUTANTS DE BOÎTE TATA ET DE BOÎTE CAAT SÉLECTIFS DE TUMEURS

(30) Priority: 30.01.2017 US 201762452075 P
(43) Date of publication of application: 04.12.2019
(73) Proprietor: EpicentRx, Inc., La Jolla, California 92037 (US)
(72) Inventor: REID, Tony, R., San Diego CA 92130 (US); ORONSKY, Bryan, T., Los Altos Hills CA 94022 (US); HEDJRAN, Farah, San Diego CA 92121 (US); LARSON, Christopher, San Diego CA 92122 (US)
(74) Representative: Graham Watt & Co LLP
(86) International application number: PCT/US2018/016025
(87) International publication number: WO 2018/140970

(56) References cited:
- EP-A1- 3 006 566
- WO-A1-2018/218240
- WO-A2-2010/086838
- WO-A2-2010/101921
- WITSCH ESTHER ET AL: "Roles for Growth Factors in Cancer Progression", PHYSIOLOGY, vol. 25, no. 2, 1 April 2010 (2010-04-01), US, pages 85 - 101, XP055891180, ISSN: 1548-9213, DOI: 10.1152/physiol.00045.2009
- DAVOLA MARIA EUGENIA ET AL: "Oncolytic viruses: how "lytic" must they be for therapeutic efficacy?", ONCOIMMUNOLOGY, vol. 8, no. 6, 28 March 2019 (2019-03-28), pages e1581528, XP055979894, Retrieved from the Internet <URL:https://tandfonline.com/doi/pdf/10.1080/2162402X.2019.1596006> DOI: 10.1080/2162402X.2019.1596006
- HEMMINKI OTTO ET AL: "Oncolytic viruses for cancer immunotherapy", JOURNAL OF HEMATOLOGY & ONCOLOGY, vol. 13, no. 1, 29 June 2020 (2020-06-29), XP093084557, Retrieved from the Internet <URL:https://link.springer.com/article/10.1186/s13045-020-00922-1/fulltext.html> DOI: 10.1186/s13045-020-00922-1
- HEDJRAN F ET AL.: "Deletion analysis of Ad5 Ela transcriptional control region: impact on tumor-selective expression of Ela and Elb", CANCER GENE THERAPY, vol. 18, 2011, pages 717 - 723, XP002679460
- HANAKA S ET AL.: "Regulation of In Vitro and In Vivo Transcription of Early-Region IV of Adenovirus Type 5 by Multiple cis-Acting Elements", MOLECULAR AND CELLULAR BIOLOGY, vol. 7, 1987, pages 2578 - 2587, XP055522845
- NISHIGAKI T ET AL.: "A Specific Domain of the Adenovirus EIV Promoter is Necessary To Maintain Susceptibility of the Integrated Promoter to EIA Transactivation", MOLECULAR AND CELLULAR BIOLOGY, vol. 8, 1988, pages 353 - 360, XP055522849

## Description

### FIELD OF THE INVENTION

The field of the invention is molecular biology and virology, specifically modified viruses that preferentially infect hyperproliferative and/or non-growth arrested cells.

### BACKGROUND

Despite extensive knowledge of the underlying molecular mechanisms that cause cancer, most advanced cancers remain incurable with current chemotherapy and radiation protocols. Oncolytic viruses have emerged as a platform technology that has the potential to significantly augment current standard treatment for a variety of malignancies (Kumar, S. et al. (2008) CURRENT OPINION IN MOLECULAR THERAPEUTICS 10(4):371-379; Kim, D. (2001) EXPERT OPINION ON BIOLOGICAL THERAPY 1(3):525-538; Kim D. (2000) ONCOGENE 19(56):6660-6669). These viruses have shown promise as oncolytic agents that not only directly destroy malignant cells via an infection-to-reproduction-to-lysis chain reaction but also indirectly induce anti-tumor immunity. These immune stimulatory properties have been augmented with the insertion of therapeutic transgenes that are copied and expressed each time the virus replicates.

Previously developed oncolytic viruses include the oncolytic serotype 5 adenovirus (Ad5) referred to as TAV-255 that is transcriptionally attenuated in normal cells but transcriptionally active in cancer cells (see, PCT Publication No. WO2010/101921). It is believed that the mechanism by which the TAV-255 vector achieves this tumor selectivity is through targeted deletion of three transcriptional factor (TF) binding sites for the transcription factors Pea3 and E2F, proteins that regulate adenovirus expression of E1a, the earliest gene to be transcribed after virus entry into the host cell, through binding to specific DNA sequences.

Despite the efforts to date, there is a need for improved oncolytic viruses that, in particular, exhibit tumor-selective replication, viral mediated lysis, and/or therapeutic transgene expression for treating cancers and hyperproliferative disorders in human patients. In the prior art, Hedjran F et al., "Deletion analysis of Ad5 Ela transcriptional control region: impact on tumor-selective expression of Ela and Elb", Cancer Gene Therapy, (20110000), vol. 18, pages 717 - 723. WO 2010/101921 A2 discloses tumor-selective E1A and E1B mutants.

### SUMMARY OF THE INVENTION

The invention is defined in the appended claims. Embodiments of the invention are provided in the dependent claims. The invention is based, in part, upon the discovery that, for certain viral promoters, the TATA and/or CAAT box, while necessary to drive transcription in normal, healthy cells, is dispensable for active transcription in cancerous cells.

Accordingly, in one aspect, the invention provides a recombinant virus comprising: (i) a modified TATA box-based promoter operably linked to a gene, wherein the modified TATA box-based promoter lacks a functional TATA box and permits selective expression of the gene in a cancer cell; and/or (ii) a modified CAAT box-based promoter operably linked to a gene, wherein the modified CAAT box-based promoter lacks a functional CAAT box and permits selective expression of the gene in a cancer cell, according to the claims.

A recombinant virus is described comprising a modified TATA box-based promoter operably linked to a gene, wherein the modified TATA box-based promoter lacks a functional TATA box and permits selective expression of the gene in a hyperproliferative and/or non-growth arrested cell.

A recombinant virus is also described comprising a modified CAAT box-based promoter operably linked to a gene, wherein the modified CAAT box-based promoter lacks a functional CAAT box and permits selective expression of the gene in a hyperproliferative and/or non-growth arrested cell.

In certain embodiments of any of the foregoing recombinant viruses, the recombinant virus is selected from a recombinant vaccinia virus, adenovirus, adeno-associated virus (AAV), herpes simplex virus 1 (HSV1), myxoma virus, reovirus, poliovirus, vesicular stomatitis virus (VSV), measles virus (MV), and Newcastle disease virus (NDV). In certain embodiments, the recombinant virus is an adenovirus, *e.g*., a type 5 adenovirus (Ad5) or a type 35 adenovirus (Ad35), *e.g.,* a type 5 adenovirus. In certain embodiments, the modified TATA box-based promoter and/or the modified CAAT box-based promoter is an early gene promoter, *e.g.,* an E1a promoter, E1b promoter, or E4 promoter, *e.g.,* an E1a promoter.

In certain embodiments of any of the foregoing recombinant viruses, the modification included in the modified TATA box-based promoter comprises a deletion of the entire TATA box. In certain embodiments, the virus comprises a deletion of nucleotides corresponding to -27 to -24, -31 to -24, -44 to +54, or -146 to +54 of the adenovirus type 5 E1a promoter, which correspond, respectively, to nucleotides 471 to 474, 467 to 474, 454 to 551 and 352 to 551 of SEQ ID NO: 2, and to nucleotides 472 to 475, 468 to 475, 455 to 552, and 353 to 552 of SEQ ID NO: 8.

In certain embodiments, the virus comprises a deletion of nucleotides corresponding to -29 to -26, -33 to -26, -44 to +52, or -148 to +52 of the adenovirus type 5 E1a promoter. In certain embodiments, the virus comprises a deletion of nucleotides corresponding to nucleotides 471 to 475, 467 to 475, 446 to 551 and 352 to 551 of SEQ ID NO: 2.

In another aspect, the invention provides a recombinant virus, wherein the virus is a type 5 adenovirus, and the virus comprises a deletion of nucleotides corresponding to -27 to - 24, -31 to -24, -44 to +54, or -146 to +54 of the adenovirus type 5 E1a promoter, which correspond, respectively, to nucleotides 471 to 474, 467 to 474, 454 to 551 and 352 to 551 of SEQ ID NO: 2, and to nucleotides 472 to 475, 468 to 475, 455 to 552, and 353 to 552 of SEQ ID NO: 8.

In another aspect, the invention provides a recombinant virus, wherein the virus is a type 5 adenovirus, and the virus comprises a deletion of nucleotides corresponding to -29 to - 26, -33 to -26, -44 to +52, or -148 to +52 of the adenovirus type 5 E1a promoter or a deletion of nucleotides corresponding to nucleotides 471 to 475, 467 to 475, 446 to 551 and 352 to 551 of SEQ ID NO: 2.

In another aspect, the invention provides a recombinant virus, wherein the virus is a type 5 adenovirus, and the virus comprises a polynucleotide deletion that results in a recombinant type 5 adenovirus comprising the sequence CTAGGACTG (SEQ ID NO: 7), AGTGCCCG (SEQ ID NO: 12), or TATTCCCG (SEQ ID NO: 13), which result from joining the two polynucleotide sequences that would otherwise flank the deleted polynucleotide sequence.

In certain embodiments of any of the foregoing recombinant viruses, the modification included in the modified CAAT box-based promoter comprises a deletion of the entire CAAT box. In certain embodiments, the virus comprises a deletion of nucleotides corresponding to -76 to -68 of the adenovirus type 5 E1a promoter, which corresponds to nucleotides 422 to 430 of SEQ ID NO: 2, and to nucleotides 423 to 431 of SEQ ID NO: 8.

In another aspect, the invention provides a recombinant virus, wherein the virus is a type 5 adenovirus, and the virus comprises a deletion of nucleotides corresponding to -76 to -68 of the adenovirus type 5 E1a promoter, which corresponds to nucleotides 422 to 430 of SEQ ID NO: 2, and to nucleotides 423 to 431 of SEQ ID NO: 8.

In certain embodiments of any of the foregoing recombinant viruses, the virus comprises the nucleotide sequence of SEQ ID NO: 3, SEQ ID NO: 15, SEQ ID NO: 16, SEQ ID NO: 17, SEQ ID NO: 20, SEQ ID NO: 21, SEQ ID NO: 22, or SEQ ID NO: 23, or a sequence having 80%, 85%, 86%, 87%, 88%, 89%, 90%, 91%, 92%, 93%, 94%, 95%, 96%, 97%, 98%, or 99% sequence identity to SEQ ID NO: 3, SEQ ID NO: 15, SEQ ID NO: 16, SEQ ID NO: 17, SEQ ID NO: 20, SEQ ID NO: 21, SEQ ID NO: 22, or SEQ ID NO: 23.

In another aspect, the invention provides a recombinant virus, wherein the virus is a type 5 adenovirus, and the virus comprises a polynucleotide deletion that results in a recombinant type 5 adenovirus comprising the sequence TTCCGTGGCG (SEQ ID NO: 14), which results from joining the two polynucleotide sequences that would otherwise flank the deleted polynucleotide sequence.

In certain embodiments of any of the foregoing recombinant viruses, the virus comprises a deletion of nucleotides corresponding to 477 to 484 of the Ad35 genome.

In certain embodiments, any of the foregoing recombinant viruses may further comprise a nucleotide sequence encoding a therapeutic transgene. The therapeutic transgene may encode a therapeutic polypeptide, *e.g.,* an apoptotic agent, antibody, CTL responsive peptide, cytokine, cytolytic agent, cytotoxic agent, enzyme, heterologous antigen expressed on the surface of a tumor cell to elicit an immune response, immunostimulatory or immunomodulatory agent, interferon, lytic peptide, oncoprotein, polypeptide which catalyzes processes leading to cell death, polypeptide which complements genetic defects in somatic cells, tumor suppressor protein, vaccine antigen, and any combination thereof. The therapeutic transgene may encode a therapeutic nucleic acid, *e.g.,* an antisense RNA or a ribozyme. In certain embodiments, the therapeutic transgene is selected from acetylcholine, an anti-PD-1 antibody heavy chain or light chain, an anti-PD-L1 antibody heavy chain or light chain, BORIS/CTCFL, CD19, CD20, CD80, CD86, CD137L, CD154, DKK1/Wnt, ICAM-1, IL-1, IL-3, IL-4, IL-5, IL-6, IL-8, IL-9, IL-17, IL-23, IL-23A/p19, interferon-gamma, TGF-β, a TGF-β trap, FGF, IL-24, IL-27, IL-35, MAGE, NY-ESO-1, p53, and thymidine kinase. In certain embodiments, the therapeutic transgene is a TGF-β trap. In certain embodiments, the recombinant virus comprises an E1b-19K and an E1b-55K start site, and the nucleotide sequence encoding the therapeutic transgene is inserted between the start site of E1b-19K and the start site of E1b-55K.

In certain embodiments, any of the foregoing recombinant viruses may comprise a deletion of at least one Pea3 binding site, or a functional portion thereof.

In certain embodiments, any of the foregoing recombinant viruses may selectively replicate in a hyperproliferative cell and/or a non-growth arrested cell. In certain embodiments, any of the foregoing recombinant viruses may selectively express E1a, E1b, and/or a therapeutic transgene in a hyperproliferative cell and/or a non-growth arrested cell. In certain embodiments, any of the foregoing recombinant viruses may selectively have cytolytic activity in a hyperproliferative cell and/or a non-growth arrested cell.

The hyperproliferative and/or non-growth arrested cell may be a cancer cell, endothelial cell, epidermal cell, fibroblast, and/or immune cell. The hyperproliferative and/or non-growth arrested cell may be a cancer cell, *e.g*., an anal cancer, basal cell carcinoma, bladder cancer, bone cancer, brain cancer, breast cancer, carcinoma, cholangiocarcinoma, cervical cancer, colon cancer, colorectal cancer, endometrial cancer, gastroesophageal cancer, gastrointestinal (GI) cancer, gastrointestinal stromal tumor, hepatocellular carcinoma, gynecologic cancer, head and neck cancer, hematologic cancer, kidney cancer, leukemia, liver cancer, lung cancer, lymphoma, melanoma, merkel cell carcinoma, mesothelioma, neuroendocrine cancer, non-small cell lung cancer, ovarian cancer, pancreatic cancer, pediatric cancer, prostate cancer, renal cell carcinoma, sarcoma, skin cancer, small cell lung cancer, squamous cell carcinoma of the skin, stomach cancer, testicular cancer or thyroid cancer cell.

A recombinant virus comprising any modified or deleted viral regulatory sequence that permits selective expression of the virus in a hyperproliferative and/or non-growth arrested cell is described.

In another aspect, the invention provides a pharmaceutical composition comprising any one or a combination of the foregoing recombinant viruses and at least one pharmaceutically acceptable carrier or diluent.

A method of treating a hyperproliferative disease, in a subject is described. The method comprises administering to the subject an effective amount of a recombinant virus described herein to treat the hyperproliferative disease in the subject. In certain embodiments, the hyperproliferative disease is selected from cancer, atherosclerosis, rheumatoid arthritis, psoriasis, lupus, idiopathic pulmonary fibrosis, sclerodermapulmonary hypertension, asthma, kidney fibrosis, COPD, cystic fibrosis, DIP, UIP, macular degeneration, restenosis, retinopathies, hyperproliferative fibroblast disorders, scleroderma, glomerulonephritis, diabetic nephropathy, malignant nephrosclerosis, thrombotic microangiopathy syndromes, transplant rejection, glomerulopathies and cirrhosis.

In the invention, the hyperproliferative disease is cancer. In certain embodiments, the cancer is selected from anal cancer, basal cell carcinoma, bladder cancer, bone cancer, brain cancer, breast cancer, carcinoma, cholangiocarcinoma, cervical cancer, colon cancer, colorectal cancer, endometrial cancer, gastroesophageal cancer, gastrointestinal (GI) cancer, gastrointestinal stromal tumor, hepatocellular carcinoma, gynecologic cancer, head and neck cancer, hematologic cancer, kidney cancer, leukemia, liver cancer, lung cancer, lymphoma, melanoma, merkel cell carcinoma, mesothelioma, neuroendocrine cancer, non-small cell lung cancer, ovarian cancer, pancreatic cancer, pediatric cancer, prostate cancer, renal cell carcinoma, sarcoma, skin cancer, small cell lung cancer, squamous cell carcinoma of the skin, stomach cancer, testicular cancer and thyroid cancer.

A method of inhibiting tumor growth in a subject is described. The method comprises administering to the subject an effective amount of a recombinant virus described herein to inhibit proliferation of the tumor cell.

A method of inhibiting proliferation of a tumor cell is described. The method comprises exposing the cell to an effective amount of a recombinant viruses described herein to inhibit proliferation of the tumor cell.

In each of the foregoing methods, the recombinant virus can, *e.g*., be administered in combination with one or more therapies selected from surgery, radiation, chemotherapy, immunotherapy, hormone therapy, and virotherapy. In each of the foregoing methods, the effective amount of the recombinant virus can comprise, *e.g.*, 10²-10¹⁵ plaque forming units (pfus). In each of the foregoing methods, the subject can, *e.g.,* be a human, *e.g.,* a pediatric human, or an animal.

In each of the foregoing methods, the effective amount of the recombinant virus may, *e.g.,* be identified by measuring an immune response to an antigen in the subject. In certain embodiments, the immune response to the antigen is measured by injecting the subject with the antigen at an injection site on the skin of the subject and measuring the size of an induration at the injection site.

A method of expressing a therapeutic transgene in a target cell is described. The method comprises exposing the cell to an effective amount of the recombinant virus described herein to express the target transgene.

A method of engineering an oncolytic virus is also described. The method comprises modifying a viral TATA box-based promoter operably linked to a gene such that the modified TATA box-based promoter lacks a functional TATA box and permits selective expression of the gene in a cancer cell.

A method of engineering an oncolytic virus is also described. The method comprises modifying a viral CAAT box-based promoter operably linked to a gene such that the modified CAAT box-based promoter lacks a functional CAAT box and permits selective expression of the gene in a cancer cell.

In another aspect, the invention provides a method of engineering an oncolytic virus according to the claims. The method comprises modifying a viral TATA box-based promoter operably linked to a gene such that the modified TATA box-based promoter lacks a functional TATA box and permits selective expression of the gene in a cancer cell and/or modifying a viral CAAT box-based promoter operably linked to a gene such that the modified CAAT box-based promoter lacks a functional CAAT box and permits selective expression of the gene in a cancer cell.

In another aspect, the invention provides an isolated nucleic acid comprising a nucleotide sequence of SEQ ID NO: 3, SEQ ID NO: 15, SEQ ID NO: 16, SEQ ID NO: 17, SEQ ID NO: 20, SEQ ID NO: 21, SEQ ID NO: 22, or SEQ ID NO: 23. A sequence having 80%, 85%, 86%, 87%, 88%, 89%, 90%, 91%, 92%, 93%, 94%, 95%, 96%, 97%, 98%, or 99% sequence identity to SEQ ID NO: 3, SEQ ID NO: 15, SEQ ID NO: 16, SEQ ID NO: 17, SEQ ID NO: 20, SEQ ID NO: 21, SEQ ID NO: 22, or SEQ ID NO: 23 is described. In certain embodiments, the isolated nucleic acid comprises the nucleotide sequence of SEQ ID NO: 4. The invention provides host cells comprising one or more of the foregoing nucleic acids.

In another aspect, the invention provides a method of producing a recombinant virus. The method comprises: (a) growing one or more of the foregoing host cells under conditions so that the host cell produces the recombinant virus; and (b) purifying the recombinant virus.

These and other aspects and advantages of the invention are illustrated by the following figures, detailed description and claims.

### DESCRIPTION OF THE DRAWINGS

The invention can be more completely understood with reference to the following drawings.

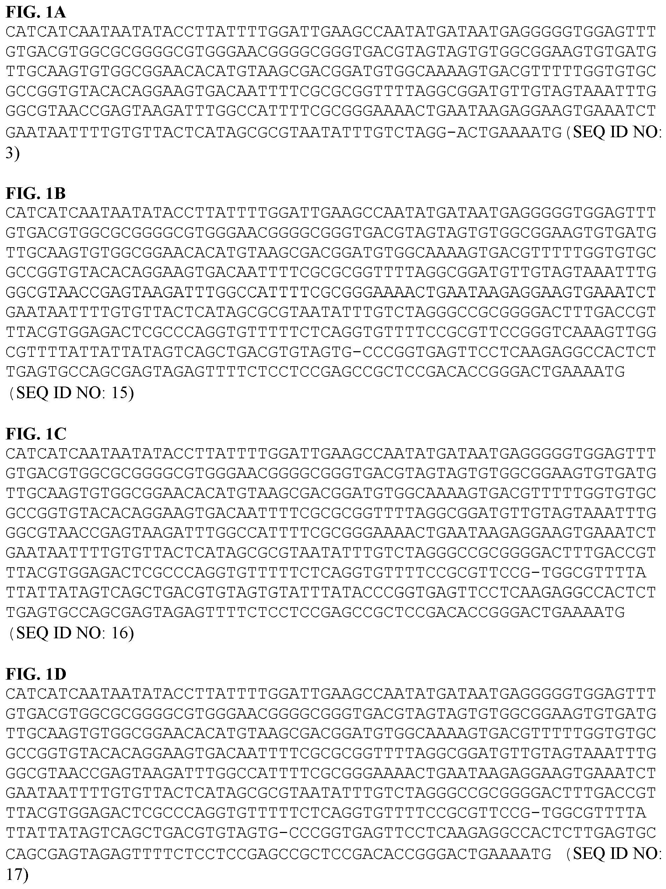

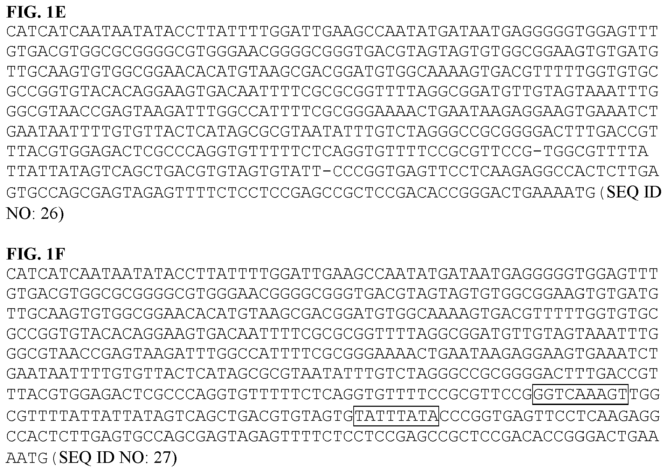

**FIG. 1A** depicts the nucleotide sequence of the 5' end of Ad-Δ350 (which includes deletions of both the TATA box and the CAAT box) up to the start codon of the E1a gene. The site of the 200 nucleotide deletion from the wild-type adenoviral sequence is denoted with a hyphen. **FIG. 1B** depicts the nucleotide sequence of the 5' end of Ad-TATA up to the start codon of the E1a gene. The site of the 8 nucleotide deletion from the wild-type adenoviral sequence is denoted with a hyphen. **FIG. 1C** depicts the nucleotide sequence of the 5' end of Ad-CAAT up to the start codon of the E1a gene. The site of the 9 nucleotide deletion from the wild-type adenoviral sequence is denoted with a hyphen. **FIG. 1D** depicts the nucleotide sequence of the 5' end of Ad-CAAT-TATA up to the start codon of the E1a gene. The site of the 9 nucleotide and 8 nucleotide deletions from the wild-type adenoviral sequence are denoted with hyphens. **FIG. 1E** depicts the nucleotide sequence of the 5' end of Ad-CAAT-mTATA up to the start codon of the E1a gene. The site of the 9 nucleotide and 4 nucleotide deletions from the wild-type adenoviral sequence are denoted with hyphens. **FIG. 1F** depicts the nucleotide sequence of the 5' end of wild-type Ad5 up to the start codon of the E1a gene. The CAAT box (GGTCAAAGT) and TATA box (TATTTATA) are indicated with boxes.
**FIG. 2A** depicts a Western blot showing E1a expression levels in cancerous Panc-1 cells at the indicated hours following infection with Ad-Δ350 or Ad-TAV-255. **FIG. 2B** depicts a Western blot showing E1a expression levels in non-cancerous WI-38 cells at the indicated hours following the infection with Ad-Δ350 or Ad-TAV-255. L represents ladder and CN represents non-infected control.
**FIG. 3A** depicts a Western blot showing E1a expression levels in cancerous Panc-1 cells 72 hours following infection with Ad-Δ350 or Ad-TAV-255 at a multiplicity of infection (MOI) of 3 or 5. **FIG. 3B** depicts a Western blot showing E1a expression levels in cancerous A549 cells 72 hours following infection with Ad-Δ350 or Ad-TAV-255 at a multiplicity of infection (MOI) of 3 or 5. L represents ladder and CN represents non-infected control.
**FIG. 4A** depicts crystal violet staining of cancerous HCT116 cells, Panc-1 cells, and A549 cells at the indicated time points following infection with Ad-Δ350 at the indicated MOI. **FIG. 4B** depicts crystal violet staining of non-cancerous MRC5 cells and WI38 cells 10 days following infection with Ad-Δ350 or Ad-TAV-255 at the indicated MOI. Crystal violet stains viable cells blue. CN represents non-infected control.
**FIG. 5** depicts crystal violet staining of cancerous A549, Panc1, HCT116, and Hep3b cells as non-infected controls and three days after infection with Ad-CAAT or Ad-CAAT-mTATA at 5 MOI. Crystal violet stains viable cells blue.
**FIG. 6** depicts crystal violet staining of cancerous ADS-12, ASPC1, HT-29, and Hep3b cells as non-infected controls and three days after infection with Ad-TATA, Ad-CAAT, Ad-CAAT-TATA, Ad-Δ350, and Ad-TAV-Δ19k at 5 MOI. Crystal violet stains viable cells blue.
**FIG. 7** depicts crystal violet staining of cancerous ADS-12, ASPC1, HT-29, and Hep3b cells as non-infected controls and four days after infection with Ad-TATA, Ad-CAAT, Ad-CAAT-TATA, Ad-Δ350, and Ad-TAV-Δ19k at 5 MOI. Crystal violet stains viable cells blue.
**FIG. 8** depicts crystal violet staining of cancerous Panc1, A549, MeWo, and HCT-116 cells as non-infected controls and three days after infection with Ad-TATA, Ad-CAAT, Ad-CAAT-TATA, Ad-Δ350, and Ad-TAV-Δ19k at 5 MOI. Crystal violet stains viable cells blue.
**FIG. 9** depicts crystal violet staining of cancerous Panc1, A549, MeWo, and HCT-116 cells as non-infected controls and four days after infection with Ad-TATA, Ad-CAAT, Ad-CAAT-TATA, Ad-Δ350, and Ad-TAV-Δ19k at 5 MOI. Crystal violet stains viable cells blue.
**FIG. 10** depicts crystal violet staining of cancerous A549, HCT116, Hep3b, and Panc1 cells as non-infected controls and five days after infection with Ad-TATA, Ad-CAAT, Ad-CAAT-TATA, Ad-Δ350, and Ad-TAV-Δ19k at 5 MOI. Crystal violet stains viable cells blue.
**FIG. 11** depicts crystal violet staining of cancerous MeWo, HT29, ADS12, and ASPC cells as non-infected controls and five days after infection with Ad-TATA, Ad-CAAT, Ad-CAAT-TATA, Ad-Δ350, and Ad-TAV-Δ19k at 5 MOI. Crystal violet stains viable cells blue.
**FIG. 12** depicts crystal violet staining of non-cancerous WI38 cells as non-infected controls and four days after infection with Ad-TATA, Ad-CAAT, Ad-CAAT-TATA, Ad-Δ350, and Ad-TAV-Δ19k at the indicated MOI. Crystal violet stains viable cells blue.
**FIG. 13** depicts crystal violet staining of non-cancerous WI38 cells as non-infected controls and six days after infection with Ad-TATA, Ad-CAAT, Ad-CAAT-TATA, Ad-Δ350, and Ad-TAV-Δ19k at the indicated MOI. Crystal violet stains viable cells blue.
**FIG. 14** depicts crystal violet staining of cancerous Panc-1 cells, A549 cells, and ADS12 cells five days after infection with Ad-Δ350-Δ19k at the indicated MOI. Crystal violet stains viable cells blue. CN represents non-infected control.
**FIG. 15** depicts crystal violet staining of cancerous Panc-1 cells, A549 cells, and ADS12 cells five days after infection with Ad-Δ350-GM-CSF at the indicated MOI. Crystal violet stains viable cells blue. CN represents non-infected control.
**FIG. 16** depicts crystal violet staining of cancerous A549 cells three days after infection with Ad-Δ350-Δ19k, Ad-Δ350-mGM-CSF, and Ad-TAV-Δ19k at 5 MOI. Crystal violet stains viable cells blue. CN represents non-infected control.
**FIG. 17** depicts crystal violet staining of cancerous A549 cells five days after infection with Ad-A350-A19k, Ad-Δ350-mGM-CSF, and Ad-TAV-Δ19k at 5 MOI. Crystal violet stains viable cells blue. CN represents non-infected control.
**FIG. 18** depicts crystal violet staining of cancerous HCT116 cells three days after infection with Ad-Δ350-Δ19k, Ad-Δ350-mGM-CSF, and Ad-TAV-Δ19k at 5 MOI. Crystal violet stains viable cells blue. CN represents non-infected control.
**FIG. 19** depicts crystal violet staining of cancerous HCT116 cells five days after infection with Ad-Δ350-Δ19k, Ad-Δ350-mGM-CSF, and Ad-TAV-Δ19k at 5 MOI. Crystal violet stains viable cells blue. CN represents non-infected control.
**FIG. 20** depicts crystal violet staining of cancerous Hep3b cells three days after infection with Ad-Δ350-Δ19k, Ad-Δ350-mGM-CSF, and Ad-TAV-Δ19k at 5 MOI. Crystal violet stains viable cells blue. CN represents non-infected control.
**FIG. 21** depicts crystal violet staining of cancerous MeWo cells five days after infection with Ad-Δ350-Δ19k, Ad-Δ350-mGM-CSF, and Ad-TAV-Δ19k at 5 MOI. Crystal violet stains viable cells blue. CN represents non-infected control.
**FIG. 22** depicts a bar graph showing mGM-CSF expression as assayed by ELISA following infection of A549 cells with Ad-Δ350-Δ19k or Ad-Δ350-mGM-CSF at 10 MOI.
**FIG. 23** depicts a bar graph showing mGM-CSF expression as assayed by ELISA following infection of ADS12 cells with Ad-Δ350-Δ19k or Ad-Δ350-mGM-CSF at the indicated MOI.
**FIG. 24** depicts tumor volumes of mice carrying subcutaneous ADS-12 tumors that were treated with three intratumoral injections of either buffer, Ad-Δ350-Δ19k (denoted 350-19k), or Ad-TAV-Δ19k (denoted TAV-19k). Each line in the figure represents the tumor volume of an individual mouse.
**FIG. 25** is an image depicting the viral cytopathic effect arising from HEK-293 cells transfected with a human adenovirus type 35 genome including a deletion of the TATA box in the E1A promoter.

### DETAILED DESCRIPTION

Transcription requires the correct positioning of RNA polymerase II (RNA pol II) on a short sequence of DNA called a promoter. A promoter sequence frequently includes a highly conserved A/T-rich sequence called a TATA box, often flanked by G/C-rich sequences, located approximately 30 base pairs upstream of the start site of transcription. Genes that lack an identifiable TATA box are typically housekeeping genes, and depend upon the transcription factor Sp1 for transcription, whereas genes containing a TATA box are typically highly regulated genes that respond to biologic response pathways. The TATA box is recognized by Transcription Factor IIB (TFIIB) and the TATA binding protein (TBP), which are required for the recruitment of RNA pol II. The central role of the TATA box in transcription is supported by experimental observations of impaired or inactivated transcription following the mutation or removal of a TATA box, *e.g.,* the removal of the TATA box in the promoter of the adenoviral E1a gene (Wu et al. (1987) NATURE 326(6112):512-5).

An additional sequence present in many promoters is a CAAT box. A CAAT box is typically located approximately 60-100 bases upstream of a gene's transcription start site and has the consensus sequence GG(T/C)CAATCT. The CAAT box is recognized by core binding factors (also referred to as nuclear factor Y or NF-Y) and CCAAT/enhancer binding proteins (C/EBPs).

The invention is based, in part, upon the discovery that for certain viral promoters, *e.g.,* the type 5 adenovirus (Ad5) E1a promoter, the TATA and/or CAAT box, while necessary to drive transcription in normal, healthy cells, is dispensable for active transcription in cancerous cells. Accordingly, in one aspect, the invention provides a recombinant virus according to the claims, comprising: (i) a modified TATA box-based promoter operably linked to a gene, wherein the modified TATA box-based promoter lacks a functional TATA box and permits selective expression of the gene in a cancer cell; and/or (ii) a modified CAAT box-based promoter operably linked to a gene, wherein the modified CAAT box-based promoter lacks a functional CAAT box and permits selective expression of the gene in a cancer cell. The TATA box-based promoter and the CAAT box-based promoter may be the same promoter *(e.g.,* the Ad5 E1a promoter), or may be different promoters.

A recombinant virus is described comprising a modified TATA box-based promoter operably linked to a gene, wherein the modified TATA box-based promoter lacks a functional TATA box and permits selective expression of the gene in a hyperproliferative and/or non-growth arrested cell.

A recombinant virus is also described comprising a modified CAAT box-based promoter operably linked to a gene, wherein the modified CAAT box-based promoter lacks a functional CAAT box and permits selective expression of the gene in a hyperproliferative and/or non-growth arrested cell.

A recombinant virus is also described comprising any modified or deleted viral regulatory sequence that permits selective expression of the virus in a hyperproliferative and/or non-growth arrested cell. Exemplary viral regulatory sequences in addition to TATA and CAAT boxes include the Ad5 E1a initiator sequence and the Ad5 E1a promoter element downstream of the TATA box.

As used herein, "TATA box" refers to a nucleotide sequence that is capable of binding to a TATA binding protein (TBP). A TATA box typically comprises an A/T-rich 8-nucleotide segment containing a core sequence of TATAAA (SEQ ID NO: 1), wherein the 8-nucleotide segment is flanked by G/C-rich sequences, however, a TATA box may bear little resemblance to the typical TATA sequence.

As used herein, a "modified TATA box" refers to a TATA box that has a deletion, substitution, or addition of one or more nucleotides relative to a wild-type TATA box sequence.

As used herein, a "functional TATA box" refers to a TATA box that is capable of binding to a TBP, *e.g.,* a TATA box that has at least 100%, at least 90%, at least 80%, at least 70%, at least 60%, at least 50%, or at least 40%, of the TBP binding activity of a corresponding wild-type TATA box sequence. As used herein, a "non-functional TATA box" refers to a TATA box that, *e.g.,* has less than 30%, less than 20%, less than 10%, or 0% of the TBP binding activity of a corresponding wild-type TATA box sequence. Assays for determining whether a TBP binds to a TATA box are known in the art. Exemplary binding assays include electrophoretic mobility shift assays, chromatin immunoprecipitation assays, and DNAse footprinting assays.

As used herein, "TATA box-based promoter" refers to any gene promoter that contains a TATA box.

As used herein, a "modified TATA box-based promoter" refers to a TATA box-based promoter that has been modified by a deletion, substitution, or addition of one or more nucleotides. In certain embodiments, the modification included in the modified TATA box-based promoter comprises a deletion of one or more nucleotides of the wild-type TATA box-based promoter sequence. In certain embodiments, the modification included in the modified TATA box-based promoter consists of a deletion of one or more nucleotides of the wild-type TATA box-based promoter sequence. In certain embodiments, the modification included in the modified TATA box-based promoter comprises a deletion of the entire TATA box of the wild-type TATA box-based promoter sequence. In certain embodiments, the modification included in the modified TATA box-based promoter consists of a deletion of the entire TATA box of the wild-type TATA box-based promoter sequence. In certain embodiments, the modification included in the modified TATA box-based promoter comprises a deletion of the entire TATA box-based promoter. In certain embodiments, the modification included in the modified TATA box-based promoter consists of a deletion of the entire TATA box-based promoter. In certain embodiments, the modification included in the modified TATA box-based promoter does not comprise an addition of or a substitution with a separate, functional promoter sequence.

In certain embodiments, the modification included in the modified TATA box-based promoter comprises a deletion of from 1 to 300, from 1 to 200, from 1 to 100, from 1 to 75, from 1 to 50, from 1 to 25, from 1 to 10, from 1 to 8, from 1 to 4 nucleotides, from 4 to 300, from 4 to 200, from 4 to 150, from 4 to 100, from 4 to 75, from 4 to 50, from 4 to 25, from 4 to 10, from 4 to 8, from 8 to 300, from 8 to 200, from 8 to 150, from 8 to 100, from 8 to 75, from 8 to 50, from 8 to 25, from 8 to 10, from 10 to 300, from 10 to 200, from 10 to 150, from 10 to 100, from 10 to 75, from 10 to 50, from 10 to 25, from 25 to 300, from 25 to 200, from 25 to 150, from 25 to 100, from 25 to 75, from 25 to 50, from 50 to 300, from 50 to 200, from 50 to 150, from 50 to 100, from 50 to 75, from 75 to 300, from 75 to 200, from 75 to 150, from 75 to 100, from 100 to 300, from 100 to 200, from 100 to 150, from 150 to 300, from 150 to 200, or from 200 to 300 nucleotides of the wild-type TATA box-based promoter sequence. In certain embodiments, the modification included in the modified TATA box-based promoter comprises a deletion of about 10, about 25, about 50, about 75, about 100, about 150, about 200, or about 300 nucleotides of the wild-type TATA box-based promoter sequence. In certain embodiments, the modification included in the modified TATA box-based promoter comprises a deletion of about 200 nucleotides of the wild-type TATA box-based promoter sequence. In certain embodiments, the modification included in the modified TATA box-based promoter comprises a deletion of 1, 2, 3, 4, 5, 6, 7, 8, or 10 nucleotides of the wild-type TATA box-based promoter sequence. In certain embodiments, the modification included in the modified TATA box-based promoter comprises a deletion of 4 or 8 nucleotides of the wild-type TATA box-based promoter sequence.

As used herein, "CAAT box" refers to a nucleotide sequence that is capable of binding to a C/EBP or NF-Y protein. A CAAT box typically comprises a consensus sequence of GG(T/C)CAATCT.

As used herein, a "modified CAAT box" refers to a CAAT box that has a deletion, substitution, or addition of one or more nucleotides relative to a wild-type CAAT box sequence.

As used herein, a "functional CAAT box" refers to a CAAT box that is capable of binding to a C/EBP or NF-Y protein, *e.g.,* a CAAT box that has at least 100%, at least 90%, at least 80%, at least 70%, at least 60%, at least 50%, or at least 40%, of the a C/EBP or NF-Y binding activity of a corresponding wild-type CAAT box sequence. As used herein, a "non-functional CAAT box" refers to a CAAT box that, *e.g.,* has less than 30%, less than 20%, less than 10%, or 0% of the a C/EBP or NF-Y binding activity of a corresponding wild-type CAAT box sequence. Assays for determining whether a C/EBP or NF-Y protein binds to a CAAT box are known in the art. Exemplary binding assays include electrophoretic mobility shift assays, chromatin immunoprecipitation assays, and DNAse footprinting assays.

As used herein, "CAAT box-based promoter" refers to any gene promoter that contains a CAAT box.

As used herein, a "modified CAAT box-based promoter" refers to a CAAT box-based promoter that has been modified by a deletion, substitution, or addition of one or more nucleotides. In certain embodiments, the modification included in the modified CAAT box-based promoter comprises a deletion of one or more nucleotides of the wild-type CAAT box-based promoter sequence. In certain embodiments, the modification included in the modified CAAT box-based promoter consists of a deletion of one or more nucleotides of the wild-type CAAT box-based promoter sequence. In certain embodiments, the modification included in the modified CAAT box-based promoter comprises a deletion of the entire CAAT box of the wild-type CAAT box-based promoter sequence. In certain embodiments, the modification included in the modified CAAT box-based promoter consists of a deletion of the entire CAAT box of the wild-type CAAT box-based promoter sequence. In certain embodiments, the modification included in the modified CAAT box-based promoter comprises a deletion of the entire CAAT box-based promoter. In certain embodiments, the modification included in the modified CAAT box-based promoter consists of a deletion of the entire CAAT box-based promoter. In certain embodiments, the modification included in the modified CAAT box-based promoter does not comprise an addition of or a substitution with a separate, functional promoter sequence.

In certain embodiments, the modification included in the modified CAAT box-based promoter comprises a deletion of from 1 to 300, from 1 to 200, from 1 to 100, from 1 to 75, from 1 to 50, from 1 to 25, from 1 to 10, from 1 to 8, from 1 to 4 nucleotides, from 4 to 300, from 4 to 200, from 4 to 150, from 4 to 100, from 4 to 75, from 4 to 50, from 4 to 25, from 4 to 10, from 4 to 8, from 8 to 300, from 8 to 200, from 8 to 150, from 8 to 100, from 8 to 75, from 8 to 50, from 8 to 25, from 8 to 10, from 10 to 300, from 10 to 200, from 10 to 150, from 10 to 100, from 10 to 75, from 10 to 50, from 10 to 25, from 25 to 300, from 25 to 200, from 25 to 150, from 25 to 100, from 25 to 75, from 25 to 50, from 50 to 300, from 50 to 200, from 50 to 150, from 50 to 100, from 50 to 75, from 75 to 300, from 75 to 200, from 75 to 150, from 75 to 100, from 100 to 300, from 100 to 200, from 100 to 150, from 150 to 300, from 150 to 200, or from 200 to 300 nucleotides of the wild-type CAAT box-based promoter sequence. In certain embodiments, the modification included in the modified CAAT box-based promoter comprises a deletion of about 10, about 25, about 50, about 75, about 100, about 150, about 200, or about 300 nucleotides of the wild-type CAAT box-based promoter sequence. In certain embodiments, the modification included in the modified CAAT box-based promoter comprises a deletion of about 200 nucleotides of the wild-type CAAT box-based promoter sequence. In certain embodiments, the modification included in the modified CAAT box-based promoter comprises a deletion of 1, 2, 3, 4, 5, 6, 7, 8, or 10 nucleotides of the wild-type CAAT box-based promoter sequence. In certain embodiments, the modification included in the modified CAAT box-based promoter comprises a deletion of 9 nucleotides of the wild-type CAAT box-based promoter sequence.

The term "operably linked" refers to a linkage of polynucleotide elements in a functional relationship. A nucleic acid sequence is "operably linked" when it is placed into a functional relationship with another nucleic acid sequence. For instance, a promoter or enhancer is operably linked to a gene if it affects the transcription of the gene. Operably linked nucleotide sequences are typically contiguous. However, as enhancers generally function when separated from the promoter by several kilobases and intronic sequences may be of variable lengths, some polynucleotide elements may be operably linked but not directly flanked and may even function *in trans* from a different allele or chromosome. In certain embodiments, a gene (coding region) is operably linked to a modified TATA box- and/or modified CAAT box-based promoter.

The term "transgene" refers to an exogenous gene or polynucleotide sequence. The term "therapeutic transgene" refers to a transgene, which when replicated and/or expressed in or by the virus imparts a therapeutic effect in a target cell, body fluid, tissue, organ, physiological system, or subject.

In certain embodiments, the recombinant virus exhibits selective expression of a gene operably linked to a modified TATA box- and/or modified CAAT box-based promoter in a hyperproliferative and/or non-growth arrested cell, *e.g.,* a cancer cell, relative to a non-hyperproliferative and/or growth arrested cell. In certain embodiments, the expression of the gene in the non-hyperproliferative and/or growth arrested cell is about 90%, about 80%, about 70%, about 60%, about 50%, about 40%, about 30%, about 20%, about 10% , or about 5% of the expression of the gene in the hyperproliferative cell and/or non-growth arrested cell. In certain embodiments, the virus exhibits no detectable expression of the gene in a non-hyperproliferative and/or growth arrested cell. In certain embodiments, the expression of a gene operably linked to a modified TATA box- and/or CAAT box-based promoter by the recombinant virus in a non-hyperproliferative and/or growth arrested cell is about 90%, about 80%, about 70%, about 60%, about 50%, about 40%, about 30%, about 20%, about 10% , or about 5% of the expression of the gene by a corresponding virus without the modified TATA box- and/or CAAT box-based promoter. In certain embodiments, the recombinant virus exhibits selective expression of an early gene, *e.g.,* adenoviral E1a or E1b. Gene expression may be determined by any appropriate method known in the art, *e.g.,* Western blot as described in Example 2 herein.

In certain embodiments, the selective expression of a gene operably linked to a modified TATA box- and/or CAAT box-based promoter, *e.g.,* an early gene, by the recombinant virus in a hyperproliferative and/or non-growth arrested cell, *e.g.,* a cancer cell, results in selective replication of the virus in the hyperproliferative and/or non-growth arrested cell. In certain embodiments, the replication of the virus in a non-hyperproliferative and/or growth arrested cell is about 90%, about 80%, about 70%, about 60%, about 50%, about 40%, about 30%, about 20%, about 10% , or about 5% of the replication of the virus in a hyperproliferative and/or non-growth arrested cell. In certain embodiments, the replication of the virus in a non-hyperproliferative and/or growth arrested cell is about 90%, about 80%, about 70%, about 60%, about 50%, about 40%, about 30%, about 20%, about 10% , or about 5% of the replication of a corresponding virus without a modified TATA box- and/or CAAT box-based promoter. Viral replication may be determined by any appropriate method known in the art, *e.g.,* by assaying the expression of viral proteins, *e.g.,* by Western blot as described in Example 2 herein, by assaying viral mediated lysis, *e.g.,* by crystal violet staining as described in Example 3 herein, or by quantitative polymerase chain reaction (qPCR).

In certain embodiments, the selective expression of a gene operably linked to a modified TATA box- and/or CAAT box-based promoter, *e.g.,* an early gene, by the recombinant virus in a hyperproliferative and/or non-growth arrested cell, *e.g.,* a cancer cell, results in selective viral mediated lysis *(i.e.,* cytolytic activity) of the hyperproliferative and/or non-growth arrested cell. In certain embodiments, the viral mediated lysis of a non-hyperproliferative and/or growth arrested cell is about 90%, about 80%, about 70%, about 60%, about 50%, about 40%, about 30%, about 20%, about 10% , or about 5% of the viral mediated lysis of a hyperproliferative and/or non-growth arrested cell. In certain embodiments, the virus exhibits no detectable viral mediated lysis of a non-hyperproliferative and/or growth arrested cell. In certain embodiments, the viral mediated lysis of a non-hyperproliferative and/or growth arrested cell is about 90%, about 80%, about 70%, about 60%, about 50%, about 40%, about 30%, about 20%, about 10% , or about 5% of the viral mediated lysis of the cell by a corresponding virus without a modified TATA box- and/or CAAT box-based promoter. Viral mediated lysis may be determined by any appropriate method known in the art, *e.g.,* crystal violet staining as described in Example 3 herein.

In certain embodiments, the selective expression of a gene operably linked to a modified TATA box- and/or CAAT box-based promoter, *e.g.,* an early gene, by the recombinant virus in a hyperproliferative and/or non-growth arrested cell, *e.g.,* a cancer cell, results in selective expression of a therapeutic transgene by the recombinant virus. In certain embodiments, the expression of a therapeutic transgene in a non-hyperproliferative and/or growth arrested cell is about 90%, about 80%, about 70%, about 60%, about 50%, about 40%, about 30%, about 20%, about 10%, or about 5% of the expression of the therapeutic transgene in the hyperproliferative and/or non-growth arrested cell. In certain embodiments, the virus exhibits no detectable expression of the therapeutic transgene in a non-hyperproliferative and/or growth arrested cell. In certain embodiments, the expression of a therapeutic transgene in a non-hyperproliferative and/or growth arrested cell is about 90%, about 80%, about 70%, about 60%, about 50%, about 40%, about 30%, about 20%, about 10% , or about 5% of the expression of the therapeutic transgene in the cell by a corresponding virus without a modified TATA box- and/or CAAT box-based promoter. Therapeutic transgene expression may be determined by any appropriate method known in the art, *e.g.,* ELISA as described in Example 4 herein.

The hyperproliferative and/or non-growth arrested cell may be a cancer cell, endothelial cell, epidermal cell, fibroblast, and/or immune cell. The hyperproliferative and/or non-growth arrested cell may be a cancer cell, *e.g.,* an anal cancer, basal cell carcinoma, bladder cancer, bone cancer, brain cancer, breast cancer, carcinoma, cholangiocarcinoma, cervical cancer, colon cancer, colorectal cancer, endometrial cancer, gastroesophageal cancer, gastrointestinal (GI) cancer, gastrointestinal stromal tumor, hepatocellular carcinoma, gynecologic cancer, head and neck cancer, hematologic cancer, kidney cancer, leukemia, liver cancer, lung cancer, lymphoma, melanoma, merkel cell carcinoma, mesothelioma, neuroendocrine cancer, non-small cell lung cancer, ovarian cancer, pancreatic cancer, pediatric cancer, prostate cancer, renal cell carcinoma, sarcoma, skin cancer, small cell lung cancer, squamous cell carcinoma of the skin, stomach cancer, testicular cancer or thyroid cancer cell. In further embodiments, the hyperproliferative cell is derived from a hyperproliferative disorder. Exemplary hyperproliferative disorders include blood vessel proliferation disorders *(e.g.,* restenosis, retinopathies, and atherosclerosis), fibrotic disorders *(e.g.,* cirrhosis, *e.g.,* hepatic cirrhosis (which may be secondary to a viral infection such as hepatitis)), mesangial disorders *(e.g.,* human renal diseases, *e.g.,* glomerulonephritis, diabetic nephropathy, malignant nephrosclerosis, thrombotic microangiopathy syndromes, transplant rejection, and glomerulopathies), precancerous disorders (e.g., hyperplasia or dysplasia), autoimmune disorders, rheumatoid arthritis, psoriasis, lupus, idiopathic pulmonary fibrosis, sclerodermapulmonary hypertension, asthma, kidney fibrosis, COPD, cystic fibrosis, DIP, UIP, macular degeneration, hyperproliferative fibroblast disorders, and scleroderma.

Sequence identity may be determined in various ways that are within the skill in the art, e.g., using publicly available computer software such as BLAST, BLAST-2, ALIGN or Megalign (DNASTAR) software. BLAST (Basic Local Alignment Search Tool) analysis using the algorithm employed by the programs blastp, blastn, blastx, tblastn and tblastx (Karlin et al., (1990) PROC. NATL. ACAD. SCI. USA 87:2264-2268; Altschul, (1993) J. MOL. EVOL. 36, 290-300; Altschul et al., (1997) NUCLEIC ACIDS RES. 25:3389-3402) are tailored for sequence similarity searching. For a discussion of basic issues in searching sequence databases see Altschul et al., (1994) NATURE GENETICS 6:119-129. Those skilled in the art can determine appropriate parameters for measuring alignment, including any algorithms needed to achieve maximal alignment over the full length of the sequences being compared. The search parameters for histogram, descriptions, alignments, expect *(i.e.,* the statistical significance threshold for reporting matches against database sequences), cutoff, matrix and filter are at the default settings. The default scoring matrix used by blastp, blastx, tblastn, and tblastx is the BLOSUM62 matrix (Henikoff et al., (1992) PROC. NATL. ACAD. Sci. USA 89:10915-10919). Four blastn parameters may be adjusted as follows: Q=10 (gap creation penalty); R=10 (gap extension penalty); wink=1 (generates word hits at every wink.sup.th position along the query); and gapw=16 (sets the window width within which gapped alignments are generated). The equivalent Blastp parameter settings may be Q=9; R=2; wink=1; and gapw=32. Searches may also be conducted using the NCBI (National Center for Biotechnology Information) BLAST Advanced Option parameter (e.g.: -G, Cost to open gap [Integer]: default = 5 for nucleotides/ 11 for proteins; -E, Cost to extend gap [Integer]: default = 2 for nucleotides/ 1 for proteins; -q, Penalty for nucleotide mismatch [Integer]: default = -3; -r, reward for nucleotide match [Integer]: default = 1; -e, expect value [Real]: default = 10; -W, wordsize [Integer]: default = 11 for nucleotides/ 28 for megablast/ 3 for proteins; -y, Dropoff (X) for blast extensions in bits: default = 20 for blastn/ 7 for others; -X, X dropoff value for gapped alignment (in bits): default = 15 for all programs, not applicable to blastn; and -Z, final X dropoff value for gapped alignment (in bits): 50 for blastn, 25 for others). ClustalW for pairwise protein alignments may also be used (default parameters may include, *e.g*., Blosum62 matrix and Gap Opening Penalty = 10 and Gap Extension Penalty = 0.1). A Bestfit comparison between sequences, available in the GCG package version 10.0, uses DNA parameters GAP=50 (gap creation penalty) and LEN=3 (gap extension penalty) and the equivalent settings in protein comparisons are GAP=8 and LEN=2.

### 1. Viruses

The term "virus" is used herein to refer any of the obligate intracellular parasites having no protein-synthesizing or energy-generating mechanism. The viral genome may be RNA or DNA. The viruses useful in the practice of the present invention include recombinantly modified enveloped or non-enveloped DNA and RNA viruses, preferably selected from baculoviridiae, parvoviridiae, picomoviridiae, herpesviridiae, poxyiridae, or adenoviridiae. A recombinantly modified virus is referred to herein as a "recombinant virus." A recombinant virus may, *e.g.,* be modified by recombinant DNA techniques to be replication deficient, conditionally replicating, or replication competent, and/or be modified by recombinant DNA techniques to include expression of exogenous transgenes. Chimeric viral vectors which exploit advantageous elements of each of the parent vector properties (See, *e.g.,* Feng et al. (1997) NATURE BIOTECHNOLOGY 15:866-870) may also be useful in the practice of the present invention. Although it is generally favored to employ a virus from the species to be treated, in certain instances it may be advantageous to use vectors derived from different species that possess favorable pathogenic features. For example, equine herpes virus vectors for human gene therapy are described in PCT Publication No. WO 98/27216. The vectors are described as useful for the treatment of humans as the equine virus is not pathogenic to humans. Similarly, ovine adenoviral vectors may be used in human gene therapy as they are claimed to avoid the antibodies against the human adenoviral vectors. Such vectors are described in PCT Publication No. WO 97/06826.

Viruses useful for the practice of the invention contain a TATA box- and/or CAAT box-based promoter. In certain embodiments, the TATA box- and/or CAAT box-based promoter is the promoter for an early phase gene, *e.g.,* a gene encoding a protein that is produced following entry into the host cell but prior to replication, which typically initiates replication of the genome and expression of late genes.

Examples of viruses with early gene TATA box- and/or CAAT box-based promoters include Human immunodeficiency virus-1 (HIV-1), herpes viruses simplex virus type 1, adeno-associated virus, Influenza virus, reovirus, vesicular stomatitis virus (VSV), newcastle virus, vaccinia virus, poliovirus, measles virus, mumps virus, sindbis virus (SIN), and sendai virus (SV).

Preferably, the recombinant virus is an adenovirus. Adenoviruses are medium-sized (90-100 nm), non-enveloped (naked), icosahedral viruses composed of a nucleocapsid and a double-stranded linear DNA genome. Adenoviruses replicate in the nucleus of mammalian cells using the host's replication machinery. The term "adenovirus" refers to any virus in the genus Adenoviridiae including, but not limited to, human, bovine, ovine, equine, canine, porcine, murine, and simian adenovirus subgenera. In particular, human adenoviruses includes the A-F subgenera as well as the individual serotypes thereof, the individual serotypes and A-F subgenera including but not limited to human adenovirus types 1, 2, 3, 4, 4a, 5, 6, 7, 8, 9, 10, 11 (Ad11a and Ad11p), 12, 13, 14, 15, 16, 17, 18, 19, 19a, 20, 21, 22, 23, 24, 25, 26, 27, 28, 29, 30, 31, 32, 33, 34, 34a, 35, 35p, 36, 37, 38, 39, 40, 41, 42, 43, 44, 45, 46, 47, 48, and 91. Preferred are recombinant viruses derived from human adenovirus types 2, 5, and 35. Unless stated otherwise, all adenovirus type 5 nucleotide numbers are relative to the NCBI reference sequence AC_000008.1, which is depicted herein in SEQ ID NO: 8, and all adenovirus type 35 nucleotide numbers are relative to the NCBI reference sequence AC_000019.1, which is depicted herein in SEQ ID NO: 24. The sequence of an exemplary vector plasmid that encodes the 5' end of the adenovirus type 5 genome (pXC1) is depicted herein in SEQ ID NO: 2.

The adenovirus replication cycle has two phases: an early phase, during which four transcription units E1, E2, E3, and E4 are expressed, and a late phase which occurs after the onset of viral DNA synthesis when late transcripts are expressed primarily from the major late promoter (MLP). The late messages encode most of the virus's structural proteins. The gene products of E1, E2 and E4 are responsible for transcriptional activation, cell transformation, viral DNA replication, as well as other viral functions, and are necessary for viral growth.

In certain embodiments, the modified TATA box-based promoter is an adenoviral E1a, E1b or E4 promoter. In a certain embodiments, the modified TATA box-based promoter is an adenoviral E1a promoter, *e.g.*, the Ad5 E1a promoter. The modification included in the modified TATA box-based promoter may, *e.g.,* comprise a deletion of the entire E1a promoter TATA box, *e.g.*, comprise a deletion corresponding to nucleotides -27 to -24 of the Ad5 E1a promoter. In certain embodiments, the virus comprises a deletion of nucleotides corresponding to -27 to -24, -31 to -24, -44 to +54, or -146 to +54 of the Ad5 E1a promoter, which correspond, respectively, to nucleotides 471 to 474, 467 to 474, 454 to 551 and 352 to 551 of SEQ ID NO: 2, and to nucleotides 472 to 475, 468 to 475, 455 to 552, and 353 to 552 of SEQ ID NO: 8. In certain embodiments, the virus comprises a deletion of nucleotides corresponding to -29 to -26, -33 to -26, -44 to +52, or -148 to +52 of the Ad5 E1a promoter.

In certain embodiments, the virus comprises a deletion of nucleotides corresponding to about -50 to about -10, about -50 to about -20, about -50 to about -30, about -50 to about -40, about -40 to about -10, about -40 to about -20, about -40 to about -30, about -30 to about -10, about -30 to about -20, or about -20 to about -10 of the Ad5 E1a promoter.

In certain embodiments, the virus comprises a polynucleotide deletion that results in virus comprising the sequence CTAGGACTG (SEQ ID NO: 7), AGTGCCCG (SEQ ID NO: 12), or TATTCCCG (SEQ ID NO: 13), which result from joining the two polynucleotide sequences that would otherwise flank the deleted polynucleotide sequence. In certain embodiments, the virus comprises the sequence CTAGGACTG (SEQ ID NO: 7), AGTGCCCG (SEQ ID NO: 12), or TATTCCCG (SEQ ID NO: 13) or a sequence having 80%, 85%, 86%, 87%, 88%, 89%, 90%, 91%, 92%, 93%, 94%, 95%, 96%, 97%, 98%, or 99% sequence identity to CTAGGACTG (SEQ ID NO: 7), AGTGCCCG (SEQ ID NO: 12), or TATTCCCG (SEQ ID NO: 13)

In certain embodiments, the modified CAAT box-based promoter is an adenoviral E1a, E1b or E4 promoter. In a certain embodiments, the modified CAAT box-based promoter is an adenoviral E1a promoter, *e.g.,* the Ad5 E1a promoter. The modification included in the modified CAAT box-based promoter may, *e.g.,* comprise a deletion of the entire E1a promoter CAAT box, *e.g*., comprise a deletion corresponding to nucleotides -76 to -68 of the adenovirus type 5 E1a promoter, which corresponds to nucleotides 422 to 430 of SEQ ID NO: 2, and to nucleotides 423 to 431 of SEQ ID NO: 8.

In certain embodiments, the virus comprises a deletion of nucleotides corresponding to about -90 to about -50, about -90 to about -60, about -90 to about -70, about -90 to about -80, about -80 to about -50, about -80 to about -60, about -80 to about -70, about -70 to about -50, about -70 to about -60, or about -60 to about -50, of the Ad5 E1a promoter.

In certain embodiments, the virus comprises a polynucleotide deletion that results in virus comprising the sequence TTCCGTGGCG (SEQ ID NO: 14), which results from joining the two polynucleotide sequences that would otherwise flank the deleted polynucleotide sequence. In certain embodiments, the virus comprises the sequence TTCCGTGGCG (SEQ ID NO: 14) or a sequence having 80%, 85%, 86%, 87%, 88%, 89%, 90%, 91%, 92%, 93%, 94%, 95%, 96%, 97%, 98%, or 99% sequence identity to TTCCGTGGCG (SEQ ID NO: 14).

In certain embodiments, the virus comprises a deletion of nucleotides corresponding to about -200 to about +50, about -175 to about +50, about -150 to about +50, about -125 about +50, about -100 to about +50, about -75 to about +50, about -50 to about +50, about -25 to about +50, about +1 to about +50, about +25 to about +50, about -200 to about +25, about -175 to about +25, about -150 to about +25, about -125 about +25, about -100 to about +25, about -75 to about +25, about -50 to about +25, about -25 to about +25, about +1 to about +25, about -200 to about +1, about -175 to about +1, about -150 to about +1, about -125 about +1, about -100 to about +1, about -75 to about +1, about -50 to about +1, about -25 to about +1, about -200 to about -25, about -175 to about -25, about -150 to about -25, about -125 about -25, about -100 to about -25, about -75 to about -25, about -50 to about -25, about -200 to about -50, about -175 to about -50, about -150 to about -50, about -125 about -50, about -100 to about -50, about -75 to about -50, about -200 to about -75, about -175 to about -75, about -150 to about -75, about -125 about -75, about -100 to about -75, about -200 to about -100, about -175 to about -100, about -150 to about -100, about -125 about -100, about -200 to about -125, about -175 to about -125, about -150 to about -125, about -200 to about -150, about -175 to about -150, or about -200 to about -175 of the Ad5 E1a promoter.

In certain embodiments, in addition to a modified TATA box- and/or CAAT box-based promoter, the virus has one or more additional modifications to a regulatory sequence or promoter. An additional modification to a regulatory sequence or promoter comprises a deletion, substitution, or addition of one or more nucleotides compared to the wild-type sequence of the regulatory sequence or promoter. The additional modification may be adjacent to, or distal from, the modified TATA box- and/or CAAT box-based promoter.

In certain embodiments, the additional modification of a regulatory sequence or promoter comprises a modification of sequence of a transcription factor binding site to reduce affinity for the transcription factor, for example, by deleting a portion thereof, or by inserting a single point mutation into the binding site. In certain embodiments, the additional modified regulatory sequence enhances expression in cancer cells, but attenuates expression in normal cells.

In certain embodiments, the additional modification of a regulatory sequence or promoter comprises an additional modification to an E1a regulatory sequence. The E1a regulatory sequence contains five binding sites for the transcription factor Pea3, designated Pea3 I, Pea3 II, Pea3 III, Pea3 IV, and Pea3 V, where Pea3 I is the Pea3 binding site most proximal to the E1a start site, and Pea3 V is most distal. The E1a regulatory sequence also contains binding sites for the transcription factor E2F, hereby designated E2F I and E2F II, where E2F I is the E2F binding site most proximal to the E1a start site, and E2F II is more distal. From the E1a start site, the binding sites are arranged: Pea3 I, E2F I, Pea3 II, E2F II, Pea3 III, Pea3 IV, and Pea3 V.

In certain embodiments, at least one of these seven binding sites, or a functional portion thereof, is deleted. A "functional portion" is a portion of the binding site that, when deleted, decreases or even eliminates the functionality, e.g. binding affinity, of the binding site to its respective transcription factor (Pea3 or E2F) by, for example, at least 40%, 50%, 60%, 70%, 80%, 90%, 95% or 100% relative to the complete sequence. In certain embodiments, one or more entire binding sites are deleted. In certain embodiments, a functional portion of one or more binding sites is deleted. A "deleted binding site" encompasses both the deletion of an entire binding site and the deletion of a functional portion. When two or more binding sites are deleted, any combination of entire binding site deletion and functional portion deletion may be used.

In certain embodiments, at least one Pea3 binding site, or a functional portion thereof, is deleted. The deleted Pea3 binding site can be Pea3 I, Pea3 II, Pea3 III, Pea3 IV, and/or Pea3 V. In certain embodiments, the deleted Pea3 binding site is Pea3 II, Pea3 III, Pea3 IV, and/or Pea3 V. In certain embodiments, the deleted Pea3 binding site is Pea3 IV and/or Pea3 V. In certain embodiments, the deleted Pea3 binding site is Pea3 II and/or Pea3 III. In certain embodiments, the deleted Pea3 binding site is both Pea3 II and Pea3 III. In certain embodiments, the Pea3 I binding site, or a functional portion thereof, is retained.

In certain embodiments, at least one E2F binding site, or a functional portion thereof, is deleted. In certain embodiments, at least one E2F binding site, or a functional portion thereof, is retained. In certain embodiments, the retained E2F binding site is E2F I and/or E2F II. In certain embodiments, the retained E2F binding site is E2F II. In certain embodiments, the total deletion consists essentially of one or more of Pea3 II, Pea3 III, Pea3 IV, and/or Pea3 V, or functional portions thereof. In certain embodiments, the virus has a deletion of a 50 base pair region located from -305 to -255 upstream of the E1a initiation site, hereafter referred to as the TAV-255 deletion. In certain embodiments, the virus has a deletion of a 50 base pair region located from -304 to -255 upstream of the E1a initiation site, *e.g.,* corresponding to 195-244 of the Ad5 genome (SEQ ID NO: 8), hereafter referred to as the TAV-255 deletion. In certain embodiments, the TAV-255 deletion results in an E1a promoter that comprises the sequence GGTGTTTTGG (SEQ ID NO: 11).

A disclosed recombinant virus may comprise a nucleotide sequence that encodes for a therapeutic transgene. The therapeutic transgene may encode a therapeutic nucleic acid, *e.g.,* an antisense RNA or ribozyme RNA. The therapeutic transgene may encode a therapeutic peptide or polypeptide, *e.g.,* an apoptotic agent, antibody, CTL responsive peptide, cytokine, cytolytic agent, cytotoxic agent, enzyme, heterologous antigen expressed on the surface of a tumor cell to elicit an immune response, immunostimulatory or immunomodulatory agent, interferon, lytic peptide, oncoprotein, polypeptide which catalyzes processes leading to cell death, polypeptide which complements genetic defects in somatic cells, tumor suppressor protein, vaccine antigen, or any combination thereof.

In certain embodiments, the therapeutic transgene encodes a therapeutic polypeptide selected from acetylcholine, an anti-PD-1 antibody heavy chain or light chain, an anti-PD-L1 antibody heavy chain or light chain, BORIS/CTCFL, CD19, CD20, CD80, CD86, CD137L, CD154, DKK1/Wnt, ICAM-1, IL-1, IL-3, IL-4, IL-5, IL-6, IL-8, IL-9, IL-17, IL-23, IL-23A/p19, interferon-gamma, TGF-β, a TGF-β trap, FGF, IL-24, IL-27, IL-35, MAGE, NY-ESO-1, p53, and thymidine kinase. In certain embodiments, the therapeutic transgene is a TGF-β trap. TGF-β trap proteins suitable for use in the invention are described in United States Patent Application No. 15/717,199, filed September 27, 2017.

The adenoviral E1b-19k gene functions primarily as an anti-apoptotic gene and is a homolog of the cellular anti-apoptotic gene, BCL-2. Since host cell death prior to maturation of the progeny viral particles would restrict viral replication, E1b-19k is expressed as part of the E1 cassette to prevent premature cell death thereby allowing the infection to proceed and yield mature virions. Accordingly, in certain embodiments, a recombinant virus is provided that includes an E1b-19K insertion site, *e.g.,* the adenovirus has a nucleotide sequence encoding a therapeutic transgene inserted into an E1b-19K insertion site.

In certain embodiments, the E1b-19K insertion site is located between the start site of E1b-19K *(i.e.,* the nucleotide sequence encoding the start codon of E1b-19k, *e.g.,* corresponding to nucleotides 1714-1716 of SEQ ID NO: 8) and the start site of E1b-55K (*i.e*., the nucleotide sequence encoding the start codon of E1b-55k, *e.g*., corresponding to nucleotides 2019-2021 of SEQ ID NO: 8). Throughout the description and claims, an insertion between two sites, for example, an insertion between (i) a start site of a first gene *(e.g.,* E1b-19k) and a start site of a second gene, *(e.g.,* E1b-55K), (ii) a start site of a first gene and a stop site of a second gene, (iii) a stop site of a first gene and start site of a second gene, or (iv) a stop site of first gene and a stop site of a second gene, is understood to mean that all or a portion of the nucleotides constituting a given start site or a stop site surrounding the insertion may be present or absent in the final virus. Similarly, an insertion between two nucleotides is understood to mean that the nucleotides surrounding the insertion may be present or absent in the final virus.

In certain embodiments, the E1b-19K insertion site is located between the start site of E1b-19K *(i.e.,* the nucleotide sequence encoding the start codon of E1b-19k, *e.g.,* corresponding to nucleotides 1714-1716 of SEQ ID NO: 8) and the stop site of E1b-19K (*i.e*., the nucleotide sequence encoding the stop codon of E1b-19k, *e.g*., corresponding to nucleotides 2242-2244 of SEQ ID NO: 8). In certain embodiments, the E1b-19K insertion site comprises a deletion of from about 100 to about 305, about 100 to about 300, about 100 to about 250, about 100 to about 200, about 100 to about 150, about 150 to about 305, about 150 to about 300, about 150 to about 250, or about 150 to about 200 nucleotides adjacent the start site of E1b-19K. In certain embodiments, the E1b-19K insertion site comprises a deletion of about 200 nucleotides, *e.g.,* 203 nucleotides adjacent the start site of E1b-19K. In certain embodiments, the E1b-19K insertion site comprises a deletion corresponding to nucleotides 1714-1916 of the Ad5 genome (SEQ ID NO: 8), or the nucleotide sequence encoding the therapeutic transgene is inserted between nucleotides corresponding to 1714 and 1916 of the Ad5 genome (SEQ ID NO: 8). In certain embodiments, the nucleotide sequence encoding the therapeutic transgene is inserted between CTGACCTC (SEQ ID NO: 9) and TCACCAGG (SEQ ID NO: 10), *e.g.,* the recombinant adenovirus comprises, in a 5' to 3' orientation, CTGACCTC (SEQ ID NO: 9), the nucleotide sequence encoding the therapeutic transgene, and TCACCAGG (SEQ ID NO: 10). CTGACCTC (SEQ ID NO: 9) and TCACCAGG (SEQ ID NO: 10) define unique boundary sequences for the E1b-19K insertion site within the Ad5 genome (SEQ ID NO: 8). Throughout the description and claims, a deletion adjacent to a site, for example, a deletion adjacent to a start site of a gene or a deletion adjacent to a stop site of a gene, is understood to mean that the deletion may include a deletion of all, a portion, or none of the nucleotides constituting a given start site or a stop site.

In certain embodiments, in any of the foregoing viruses, the recombinant adenovirus further comprises an E4 deletion. In certain embodiments, the E4 deletion is located between the start site of E4-ORF6/7 *(i.e.,* the nucleotide sequence encoding the start codon of E4-ORF6/7, *e.g.*, corresponding to nucleotides 34075-34077 of SEQ ID NO: 23) and the right inverted terminal repeat (ITR; *e.g.*, corresponding to nucleotides 35836-35938 of SEQ ID NO: 23). In certain embodiments, the E4 deletion is located between the start site of E4-ORF6/7 and the start site of E4-ORF1 *(i.e.,* the nucleotide sequence encoding the start codon of E4-ORF1, *e.g.*, corresponding to nucleotides 35524-35526 of SEQ ID NO: 23). In certain embodiments, the E4 deletion comprises a deletion of a nucleotide sequence between the start site of E4-ORF6/7 and the start site of E4-ORF1. In certain embodiments, the E4 deletion comprises a deletion of from about 500 to about 2500, from about 500 to about 2000, from about 500 to about 1500, from about 500 to about 1000, from about 1000 to about 2500, from about 1000 to about 2000, from about 1000 to about 1500, from about 1500 to about 2500, from about 1500 to about 2000, or from about 2000 to about 2500 nucleotides. In certain embodiments, the E4 deletion comprises a deletion of from about 250 to about 1500, from about 250 to about 1250, from about 250 to about 1000, from about 250 to about 750, from about 250 to about 500, from 500 to about 1500, from about 500 to about 1250, from about 500 to about 1000, from about 500 to about 750, from 750 to about 1500, from about 750 to about 1250, from about 750 to about 1000, from about 1000 to about 1500, or from about 1000 to about 1250 nucleotides adjacent the start site of E4-ORF6/7. In certain embodiments, the E4 deletion comprises a deletion of about 1450 nucleotides adjacent the start site of E4-ORF6/7, *e.g.*, the E4 deletion comprises a deletion of about 1449 nucleotides adjacent the start site of E4-ORF6/7. In certain embodiments, the E4 deletion comprises a deletion corresponding to nucleotides 34078-35526 of the Ad5 genome (SEQ ID NO: 23).

### II. Methods of Viral Production

Methods for producing recombinant viruses of the invention are known in the art. Typically, a disclosed virus is produced in a suitable host cell line using conventional techniques including culturing a transfected or infected host cell under suitable conditions so as to allow the production of infectious viral particles. Nucleic acids encoding viral genes can be incorporated into plasmids and introduced into host cells through conventional transfection or transformation techniques. Exemplary suitable host cells for production of disclosed viruses include human cell lines such as HeLa, Hela-S3, HEK293, 911, A549, HER96, or PER-C6 cells. Specific production and purification conditions will vary depending upon the virus and the production system employed. For adenovirus, the traditional method for the generation of viral particles is co-transfection followed by subsequent *in vivo* recombination of a shuttle plasmid (usually containing a small subset of the adenoviral genome and optionally containing a potential transgene an expression cassette) and an adenoviral helper plasmid (containing most of the entire adenoviral genome).

Alternative technologies for the generation of adenovirus include utilization of the bacterial artificial chromosome (BAC) system, in vivo bacterial recombination in a recA÷ bacterial strain utilizing two plasmids containing complementary adenoviral sequences, and the yeast artificial chromosome (YAC) system.

Following production, infectious viral particles are recovered from the culture and optionally purified. Typical purification steps may include plaque purification, centrifugation, *e.g.,* cesium chloride gradient centrifugation, clarification, enzymatic treatment, *e.g.,* benzonase or protease treatment, chromatographic steps, *e.g.,* ion exchange chromatography or filtration steps.

### III. Therapeutic Compositions and Methods of Treatment

For therapeutic use, a recombinant virus is preferably is combined with a pharmaceutically acceptable carrier. As used herein, "pharmaceutically acceptable carrier" means buffers, carriers, and excipients suitable for use in contact with the tissues of human beings and animals without excessive toxicity, irritation, allergic response, or other problem or complication, commensurate with a reasonable benefit/risk ratio. The carrier(s) should be "acceptable" in the sense of being compatible with the other ingredients of the formulations and not deleterious to the recipient. Pharmaceutically acceptable carriers include buffers, solvents, dispersion media, coatings, isotonic and absorption delaying agents, and the like, that are compatible with pharmaceutical administration. The use of such media and agents for pharmaceutically active substances is known in the art.

Pharmaceutical compositions containing recombinant viruses disclosed herein can be presented in a dosage unit form and can be prepared by any suitable method. A pharmaceutical composition should be formulated to be compatible with its intended route of administration. Examples of routes of administration are intravenous (IV), intradermal, inhalation, transdermal, topical, transmucosal, and rectal administration. A preferred route of administration for fusion proteins is IV infusion. Useful formulations can be prepared by methods known in the pharmaceutical art. For example, see Remington's Pharmaceutical Sciences, 18th ed. (Mack Publishing Company, 1990). Formulation components suitable for parenteral administration include a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as EDTA; buffers such as acetates, citrates or phosphates; and agents for the adjustment of tonicity such as sodium chloride or dextrose.

For intravenous administration, suitable carriers include physiological saline, bacteriostatic water, Cremophor ELTM (BASF, Parsippany, NJ) or phosphate buffered saline (PBS). The carrier should be stable under the conditions of manufacture and storage, and should be preserved against microorganisms. The carrier can be a solvent or dispersion medium containing, for example, water, ethanol, polyol (for example, glycerol, propylene glycol, and liquid polyetheylene glycol), and suitable mixtures thereof.

Pharmaceutical formulations preferably are sterile. Sterilization can be accomplished by any suitable method, *e.g.*, filtration through sterile filtration membranes. Where the composition is lyophilized, filter sterilization can be conducted prior to or following lyophilization and reconstitution.

The term "effective amount" as used herein refers to the amount of an active component (*e*.*g*., the amount of a recombinant virus of the present invention) sufficient to effect beneficial or desired results. An effective amount can be administered in one or more administrations, applications or dosages and is not intended to be limited to a particular formulation or administration route.

In certain embodiments, a therapeutically effective amount of active component is in the range of 0.1 mg/kg to 100 mg/kg, *e.g.,* 1 mg/kg to 100 mg/kg, 1 mg/kg to 10 mg/kg. In certain embodiments, a therapeutically effective amount of a recombinant virus is in the range of 10² to 10¹⁵ plaque forming units (pfus), *e.g.,* 10² to 10¹⁰, 10² to 10⁵, 10⁵ to 101⁵, 10⁵ to 10¹⁰, or 10¹⁰ to 10¹⁵ plaque forming units. The amount administered will depend on variables such as the type and extent of disease or indication to be treated, the overall health of the patient, the *in vivo* potency of the antibody, the pharmaceutical formulation, and the route of administration. The initial dosage can be increased beyond the upper level in order to rapidly achieve the desired blood-level or tissue-level. Alternatively, the initial dosage can be smaller than the optimum, and the daily dosage may be progressively increased during the course of treatment. Human dosage can be optimized, e.g., in a conventional Phase I dose escalation study designed to run from 0.5 mg/kg to 20 mg/kg. Dosing frequency can vary, depending on factors such as route of administration, dosage amount, serum half-life of the virus, and the disease being treated. Exemplary dosing frequencies are once per day, once per week and once every two weeks. A preferred route of administration is parenteral, e.g., intravenous infusion. Formulation of virus -based drugs is within ordinary skill in the art. In certain embodiments, a recombinant virus is lyophilized, and then reconstituted in buffered saline, at the time of administration.

The recombinant viruses disclosed herein can be used to treat various medical indications. For example, the recombinant viruses can be used to treat various hyperproliferative diseases, *e.g.,* cancers. The hyperproliferative cells, *e.g.,* cancer cells, are exposed to a therapeutically effective amount of the recombinant virus so as to inhibit or reduce proliferation of the cancer cells. A method of treating a cancer in a subject is described. The method comprises administering to the subject an effective amount of a recombinant virus of the invention either alone or in a combination with another therapeutic agent to treat the cancer in the subject. In certain embodiments, administering an effective amount of a recombinant virus to a subject reduces tumor load in that subject by at least 30%, at least 40%, at least 50%, at least 60%, at least 70%, at least 80%, or at least 90%.

As used herein, "treat", "treating" and "treatment" mean the treatment of a disease in a subject, *e.g.,* in a human. This includes: (a) inhibiting the disease, *i.e.,* arresting its development; and (b) relieving the disease, *i.e.,* causing regression of the disease state. As used herein, the terms "subject" and "patient" refer to an organism to be treated by the methods and compositions described herein. Such organisms preferably include, but are not limited to, mammals (*e.g.*, murines, simians, equines, bovines, porcines, canines, felines, and the like), and more preferably includes humans.

Examples of cancers include solid tumors, soft tissue tumors, hematopoietic tumors and metastatic lesions. Examples of hematopoietic tumors include, leukemia, acute leukemia, acute lymphoblastic leukemia (ALL), B-cell, T-cell or FAB ALL, acute myeloid leukemia (AML), chronic myelocytic leukemia (CML), chronic lymphocytic leukemia (CLL), *e.g.,* transformed CLL, diffuse large B-cell lymphomas (DLBCL), follicular lymphoma, hairy cell leukemia, myelodyplastic syndrome (MDS), a lymphoma, Hodgkin's disease, a malignant lymphoma, non-Hodgkin's lymphoma, Burkitt's lymphoma, multiple myeloma, or Richter's Syndrome (Richter's Transformation). Examples of solid tumors include malignancies, *e.g.*, sarcomas, adenocarcinomas, and carcinomas, of the various organ systems, such as those affecting head and neck (including pharynx), thyroid, lung (small cell or non-small cell lung carcinoma (NSCLC)), breast, lymphoid, gastrointestinal (*e.g.*, oral, esophageal, stomach, liver, pancreas, small intestine, colon and rectum, anal canal), genitals and genitourinary tract (*e.g.,* renal, urothelial, bladder, ovarian, uterine, cervical, endometrial, prostate, testicular), CNS *(e.g.,* neural or glial cells, *e.g.,* neuroblastoma or glioma), or skin *(e.g.,* melanoma).

In certain embodiments, the cancer is selected from anal cancer, basal cell carcinoma, bladder cancer, bone cancer, brain cancer, breast cancer, carcinoma, cholangiocarcinoma, cervical cancer, colon cancer, colorectal cancer, endometrial cancer, gastroesophageal cancer, gastrointestinal (GI) cancer, gastrointestinal stromal tumor, hepatocellular carcinoma, gynecologic cancer, head and neck cancer, hematologic cancer, kidney cancer, leukemia, liver cancer, lung cancer, lymphoma, melanoma, merkel cell carcinoma, mesothelioma, neuroendocrine cancer, non-small cell lung cancer, ovarian cancer, pancreatic cancer, pediatric cancer, prostate cancer, renal cell carcinoma, sarcoma, skin cancer, small cell lung cancer, squamous cell carcinoma of the skin, stomach cancer, testicular cancer and thyroid cancer.

Additional exemplary hyperproliferative diseases include blood vessel proliferation disorders *(e.g.,* restenosis, retinopathies, and atherosclerosis), fibrotic disorders *(e.g.,* cirrhosis, *e.g.,* hepatic cirrhosis (which may be secondary to a viral infection such as hepatitis)), mesangial disorders *(e.g.,* human renal diseases, *e.g.,* glomerulonephritis, diabetic nephropathy, malignant nephrosclerosis, thrombotic microangiopathy syndromes, transplant rejection, and glomerulopathies), precancerous disorders (*e.g.*, hyperplasia or dysplasia), autoimmune disorders, rheumatoid arthritis, psoriasis, lupus, idiopathic pulmonary fibrosis, sclerodermapulmonary hypertension, asthma, kidney fibrosis, COPD, cystic fibrosis, DIP, UIP, macular degeneration, hyperproliferative fibroblast disorders, and scleroderma.

In certain embodiments, a recombinant virus is administered to the subject in combination with one or more therapies, *e.g.*, surgery, radiation, chemotherapy, immunotherapy, hormone therapy, or virotherapy.

In certain embodiments, a recombinant virus of the invention is administered in combination with a tyrosine kinase inhibitor, *e.g.*, erlotinib.

In certain embodiments, a recombinant virus of the invention is administered in combination with a checkpoint inhibitor, *e.g.,* an anti-CTLA-4 antibody, an anti-PD-1 antibody, or an anti-PD-L1 antibody. Exemplary anti-PD-1 antibodies include, for example, nivolumab (Opdivo^{®}, Bristol-Myers Squibb Co.), pembrolizumab (Keytruda^{®}, Merck Sharp & Dohme Corp.), PDR001 (Novartis Pharmaceuticals), and pidilizumab (CT-011, Cure Tech). Exemplary anti-PD-L1 antibodies include, for example, atezolizumab (Tecentriq^{®}, Genentech), duvalumab (AstraZeneca), MEDI4736, avelumab, and BMS 936559 (Bristol Myers Squibb Co.).

The term administered "in combination," as used herein, is understood to mean that two (or more) different treatments are delivered to the subject during the course of the subject's affliction with the disorder, such that the effects of the treatments on the patient overlap at a point in time. In certain embodiments, the delivery of one treatment is still occurring when the delivery of the second begins, so that there is overlap in terms of administration. This is sometimes referred to herein as "simultaneous" or "concurrent delivery." In other embodiments, the delivery of one treatment ends before the delivery of the other treatment begins. In some embodiments of either case, the treatment is more effective because of combined administration. For example, the second treatment is more effective, *e.g.,* an equivalent effect is seen with less of the second treatment, or the second treatment reduces symptoms to a greater extent, than would be seen if the second treatment were administered in the absence of the first treatment, or the analogous situation is seen with the first treatment. In certain embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

In certain embodiments, the effective amount of the recombinant virus is identified by measuring an immune response to an antigen in the subject and/or the method of treating the subject further comprises measuring an immune response to an antigen in the subject. Hyperproliferative diseases, *e.g.,* cancers, may be characterized by immunosuppression, and measuring an immune response to an antigen in the subject may be indicative of the level of immunosuppression in the subject. Accordingly, measuring an immune response to an antigen in the subject may be indicative of the efficacy of the treatment and/or the effective amount of the recombinant virus. The immune response to the antigen in the subject may be measured by any method known in the art. In certain embodiments, the immune response to the antigen is measured by injecting the subject with the antigen at an injection site on the skin of the subject and measuring the size of an induration or amount of inflammation at the injection site. In certain embodiments, the immune response to the antigen is measured by release of a cytokine from a cell of the subject (*e.g*., interferon gamma, IL-4 and/or IL-5) upon exposure to the antigen.

Throughout the description, where viruses, compositions, and systems are described as having, including, or comprising specific components, or where processes and methods are described as having, including, or comprising specific steps, it is contemplated that, additionally, there are compositions, devices, and systems of the present invention that consist essentially of, or consist of, the recited components, and that there are processes and methods according to the present invention that consist essentially of, or consist of, the recited processing steps.

In the application, where an element or component is said to be included in and/or selected from a list of recited elements or components, it should be understood that the element or component can be any one of the recited elements or components, or the element or component can be selected from a group consisting of two or more of the recited elements or components.

Further, it should be understood that elements and/or features of a virus, a composition, a system, a method, or a process described herein can be combined in a variety of ways without departing from the spirit and scope of the present invention, whether explicit or implicit herein. For example, where reference is made to a particular compound, that compound can be used in various embodiments of compositions of the present invention and/or in methods of the present invention, unless otherwise understood from the context. In other words, within this application, embodiments have been described and depicted in a way that enables a clear and concise application to be written and drawn, but it is intended and will be appreciated that embodiments may be variously combined or separated without parting from the present teachings and invention(s). For example, it will be appreciated that all features described and depicted herein can be applicable to all aspects of the invention(s) described and depicted herein.

It should be understood that the expression "at least one of" includes individually each of the recited objects after the expression and the various combinations of two or more of the recited objects unless otherwise understood from the context and use. The expression "and/or" in connection with three or more recited objects should be understood to have the same meaning unless otherwise understood from the context.

The use of the term "include," "includes," "including," "have," "has," "having," "contain," "contains," or "containing," including grammatical equivalents thereof, should be understood generally as open-ended and non-limiting, for example, not excluding additional unrecited elements or steps, unless otherwise specifically stated or understood from the context.

At various places in the present specification, viruses, compositions, systems, processes and methods, or features thereof, are disclosed in groups or in ranges. It is specifically intended that the description include each and every individual subcombination of the members of such groups and ranges. By way of other examples, an integer in the range of 1 to 20 is specifically intended to individually disclose 1, 2, 3, 4, 5, 6, 7, 8, 9, 10, 11, 12, 13, 14, 15, 16, 17, 18, 19, and 20.

Where the use of the term "about" is before a quantitative value, the present invention also includes the specific quantitative value itself, unless specifically stated otherwise. As used herein, the term "about" refers to a ±10% variation from the nominal value unless otherwise indicated or inferred.

It should be understood that the order of steps or order for performing certain actions is immaterial so long as the present invention remain operable. Moreover, two or more steps or actions may be conducted simultaneously.

The use of any and all examples, or exemplary language herein, for example, "such as" or "including," is intended merely to illustrate better the present invention and does not pose a limitation on the scope of the invention unless claimed. No language in the specification should be construed as indicating any non-claimed element as essential to the practice of the present invention.

### EXAMPLES

The following Examples are merely illustrative and are not intended to limit the scope or content of the invention in any way.

### Example 1: Plasmid and Adenovirus Construction

This Example describes the production of recombinant type 5 (Ad5) adenoviruses with deletions in the E1a promoter region that include a TATA and/or a CAAT box.

The adenoviral vector plasmid pXC1, which carries the 5' portion of the Ad5 genome, was acquired from Microbix Biosystem (Toronto, Canada). The nucleotide sequence of the pXC1 vector plasmid is depicted herein in SEQ ID NO: 2. The Ad5 genome NCBI reference sequence AC_000008.1 is depicted herein in SEQ ID NO: 8. **FIG. 1F** depicts the nucleotide sequence of the 5' end of wild-type Ad5 up to the start codon of the E1a gene indicating the location of the CAAT box and TATA box.

A modified pXC1 vector plasmid was generated that had a deletion of 200 nucleotides corresponding to nucleotides 352-551 of SEQ ID NO: 2 (which correspond to nucleotides 353-552 of SEQ ID NO: 8), which included the CAAT box and the TATA box in the E1a promoter. The mutated vector plasmid is hereafter referred to as pXC1-Δ350, and any resulting viral particles produced therefrom are hereafter referred to as Ad-Δ350. The nucleotide sequence of the 5' end of pXC1-Δ350, up to the start codon of the E1a gene, is shown in SEQ ID NO: 20. The full length nucleotide sequence of pXC1-Δ350 is shown in SEQ ID NO: 4. The nucleotide sequence of the 5' end of Ad-Δ350, up to the start codon of the E1a gene, is shown in **FIG. 1A** and SEQ ID NO: 3. The twenty-one nucleotides at the 5' terminus of the pXC1 vector plasmid (and any modified pXC1 vector plasmids) differ from the wild-type adenoviral sequence, however, these nucleotides are converted to the wild-type adenoviral sequence during the process of generating a recombinant adenovirus.

Where indicated, pXC1-Δ350 was further modified to carry a Sail site at the start site of the E1b-19k region and an XhoI site 200 base pairs 3' of the Sail site to facilitate insertion of therapeutic transgenes. The nucleotide sequence of the modified E1b-19k region is given in SEQ ID NO: 5. The resulting vector plasmid is hereafter referred to as pXC1-Δ350-Δ19k, and any resulting viral particles produced therefrom are hereafter referred to as Ad-Δ350-Δ19k.

Where indicated, the gene for murine GM-CSF was cloned into pXC1-A350-A19k in the modified E1b-19k region between the Sail and XhoI sites. The amino acid sequence for mouse GM-CSF is given in SEQ ID NO: 6. The resulting vector plasmid is hereafter referred to as pXC1-Δ350-mGM-CSF, and any resulting viral particles produced therefrom are hereafter referred to as Ad-Δ350-mGM-CSF.

An additional modified pXC1 vector plasmid was generated that had a deletion of 8 nucleotides corresponding to nucleotides 467-474 of SEQ ID NO: 2 (which correspond to nucleotides 468-475 of SEQ ID NO: 8), which included the TATA box in the E1a promoter. The mutated vector plasmid is hereafter referred to as pXC1-TATA, and any resulting viral particles produced therefrom are hereafter referred to as Ad-TATA. The nucleotide sequence of the 5' end of pXC1-TATA, up to the start codon of the E1a gene, is shown in SEQ ID NO: 21. The nucleotide sequence of the 5' end of Ad-TATA, up to the start codon of the E1a gene, is shown in **FIG. 1B** and SEQ ID NO: 15.

An additional modified pXC1 vector plasmid was generated that had a deletion of 9 nucleotides corresponding to nucleotides 422-430 of SEQ ID NO: 2 (which correspond to nucleotides 423-431 of SEQ ID NO: 8), which included the CAAT box in the E1a promoter. The mutated vector plasmid is hereafter referred to as pXC1-CAAT, and any resulting viral particles produced therefrom are hereafter referred to as Ad-CAAT. The nucleotide sequence of the 5' end of pXC1-CAAT, up to the start codon of the E1a gene, is shown in SEQ ID NO: 22. The nucleotide sequence of the 5' end of Ad- CAAT, up to the start codon of the E1a gene, is shown in **FIG. 1C** and SEQ ID NO: 16.

An additional modified pXC1 vector plasmid was generated that had a deletion of 9 nucleotides corresponding to nucleotides 422-430 of SEQ ID NO: 2 (which correspond to nucleotides 423-431 of SEQ ID NO: 8), which included the CAAT box in the E1a promoter, and a deletion of 8 nucleotides corresponding to nucleotides 467-474 of SEQ ID NO: 2 (which correspond to nucleotides 468-475 of SEQ ID NO: 8), which included the TATA box in the E1a promoter. The mutated vector plasmid is hereafter referred to as pXC1-CAAT-TATA, and any resulting viral particles produced therefrom are hereafter referred to as Ad-CAAT-TATA. The nucleotide sequence of the 5' end of pXC1-CAAT-TATA, up to the start codon of the E1a gene, is shown in SEQ ID NO: 23. The nucleotide sequence of the 5' end of Ad-CAAT-TATA, up to the start codon of the E1a gene, is shown in **FIG. 1D** and SEQ ID NO: 17.

An additional modified pXC1 vector plasmid was generated that had a deletion of 9 nucleotides corresponding to nucleotides 422-430 of SEQ ID NO: 2 (which correspond to nucleotides 423-431 of SEQ ID NO: 8), which included the CAAT box in the E1a promoter, and a deletion of 4 nucleotides corresponding to nucleotides 471-474 of SEQ ID NO: 2 (which correspond to nucleotides 472-475 of SEQ ID NO: 8), which included the four nucleotide TATA sequence of the TATA box in the E1a promoter (hereafter referred to as the minimal TATA or mTATA deletion). The mutated vector plasmid is hereafter referred to as pXC1-CAAT-mTATA, and any resulting viral particles produced therefrom are hereafter referred to as Ad-CAAT-mTATA. The nucleotide sequence of the 5' end of pXC1-CAAT-mTATA, up to the start codon of the E1a gene, is shown in SEQ ID NO: 25. The nucleotide sequence of the 5' end of Ad-CAAT-TATA, up to the start codon of the E1a gene, is shown in **FIG. 1E** and SEQ ID NO: 26.

An additional modified pXC1 vector plasmid was generated that had a deletion of 50 nucleotides corresponding to nucleotides 194-243 of SEQ ID NO: 2 (which corresponds to nucleotides 195-244 of SEQ ID NO: 8 and nucleotides -304 to -255 upstream of the E1a initiation site) which renders E1a expression cancer-selective (as previously described in U.S. Patent No. 9,073,980). The mutated vector plasmid is hereafter referred to as pXC1- TAV-255, and any resulting viral particles produced therefrom are hereafter referred to as Ad- TAV-255. Where indicated, pXC1- TAV-255 was further modified to carry a SalI site at the start site of the E1b-19k region and an XhoI site 200 base pairs 3' of the SalI site to facilitate insertion of therapeutic transgenes, as described above. The resulting vector plasmid is hereafter referred to as pXC1-TAV -Δ19k, and any resulting viral particles produced therefrom are hereafter referred to as Ad-TAV-Δ19k.

The various modified pXC1 plasmids were cotransfected with the plasmid pJM17 in HEK-293A cells to allow homologous recombination to rescue recombinant virus. Virus was collected and underwent two rounds of plaque purification and sequencing to test for presence of the corresponding deletion as necessary.

### Example 2: E1a Expression From Ad-Δ350 In Normal And Cancerous Cells

This Example describes a comparison between viral protein expression from the modified adenovirus Ad-Δ350 in cancerous and normal cells.

Panc1 cells (human pancreatic cancer cells) and WI-38 cells (non-cancerous human lung fibroblasts) were infected with Ad-Δ350 or Ad-TAV-255 viruses, prepared as described in Example 1. E1a expression was assayed by Western blot at the indicated hours after infection.

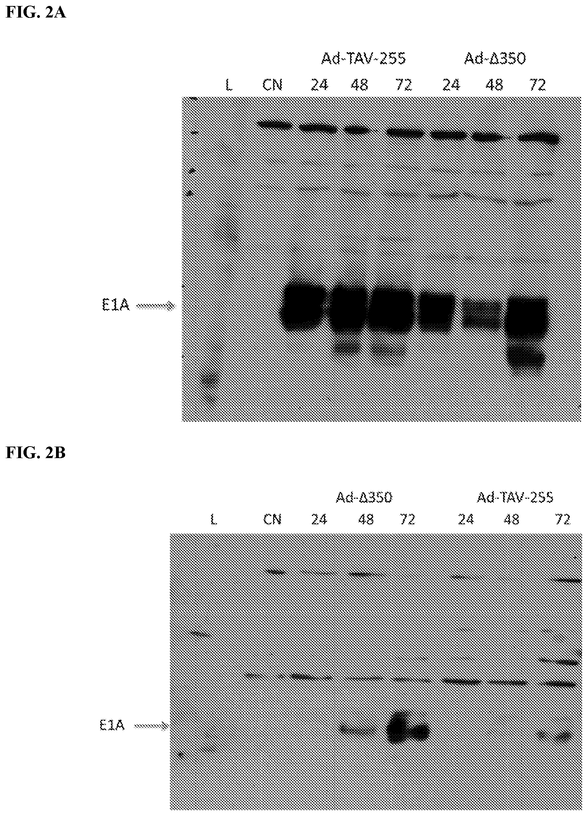

As depicted in **FIGS. 2A** and **2B****,** following infection with the Ad-Δ350 virus, WI-38 cells expressed the adenoviral protein E1a at lower levels and later time points than Panc1 cells.

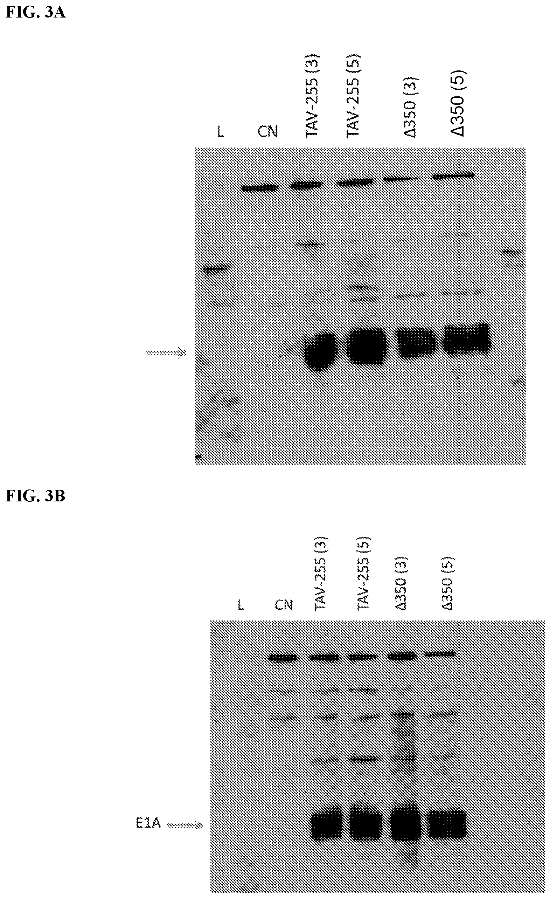

Panc1 cells and A549 cells (human lung cancer cells) were infected with Ad-TAV-255 or Ad-Δ350 at a multiplicity of infection (MOI) of 3 or 5 and E1a expression was assayed by Western blot 72 hours after infection. As depicted in **FIG. 3A** (Panc1 cells) and **FIG. 3B** (A549 cells), both cancer cell lines support high levels of E1a expression from the Ad-Δ350 or Ad-TAV-255 viruses.

Together, these results show that a 200 nucleotide region in Ad5, including the E1a TATA box, is required for E1a expression in non-cancerous cells, while this region is dispensable for E1a expression in tumor cells.

### Example 3: Cytotoxicity From Ad-Δ350, Ad-CAAT, Ad-TATA, Ad-CAAT-TATA, And Ad-CAAT-mTATA In Normal And Cancerous Cells

This Example describes a comparison between cytotoxicity resulting from the modified adenoviruses Ad-Δ350, Ad-CAAT, Ad-TATA, Ad-CAAT-TATA, and Ad-CAAT-mTATA in cancerous and normal cells.

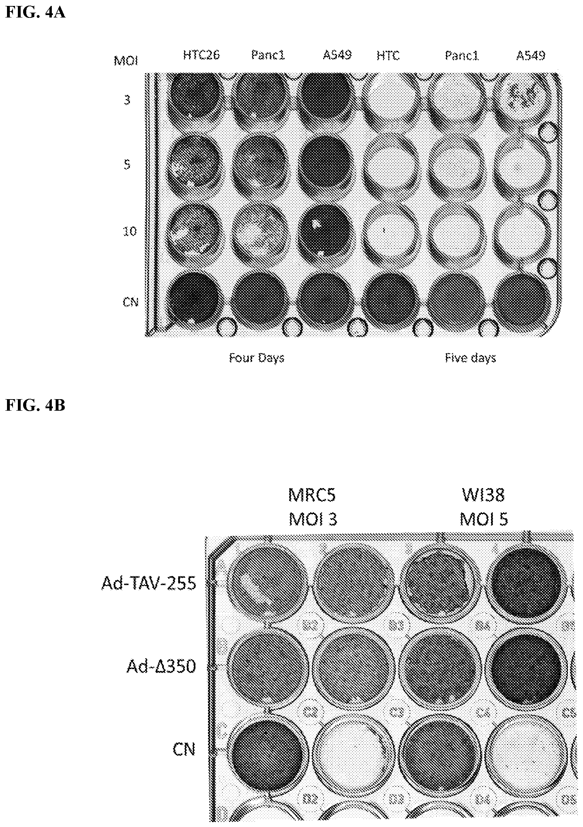

HCT116 cells (human colon cancer cells), Panc1 cells, and A549 cells were infected with Ad-Δ350, prepared as described in Example 1. Cells were infected at the indicated MOI or kept as non-infected controls and stained with crystal violet, which stains viable cells blue at the indicated times after infection. As depicted in **FIG. 4A****,** each of the cancerous cell lines showed extensive cell death from four to five days after infection.

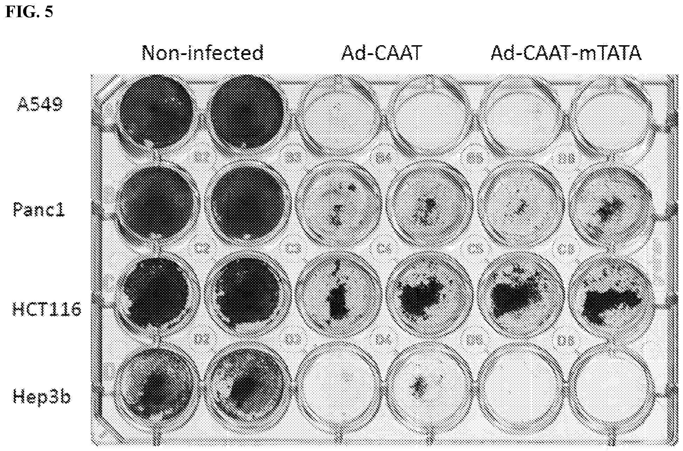

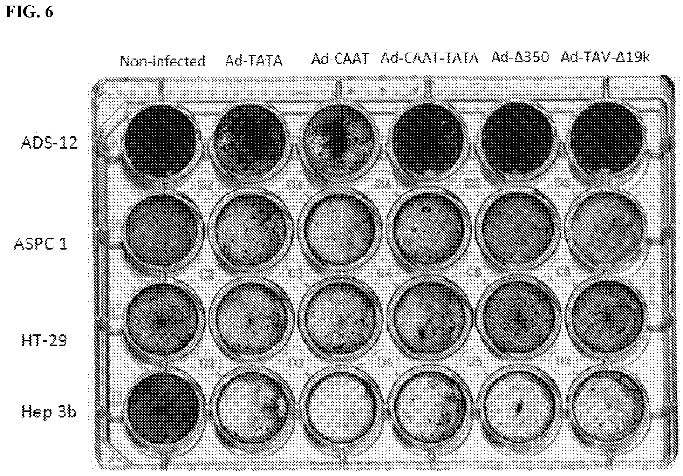

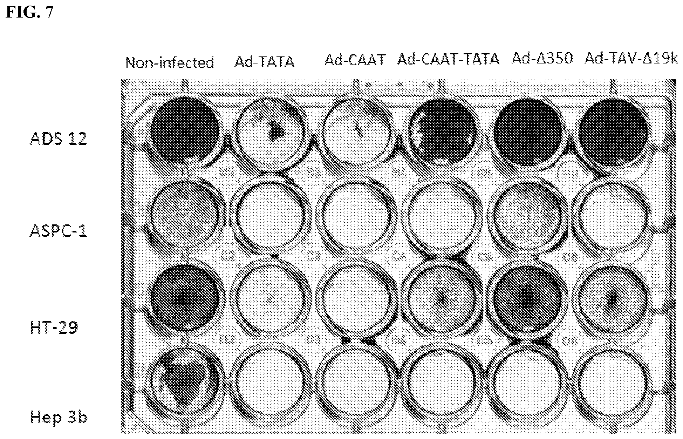

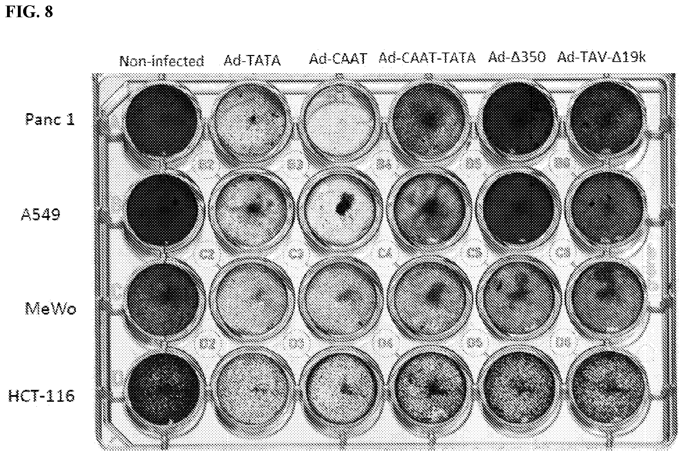

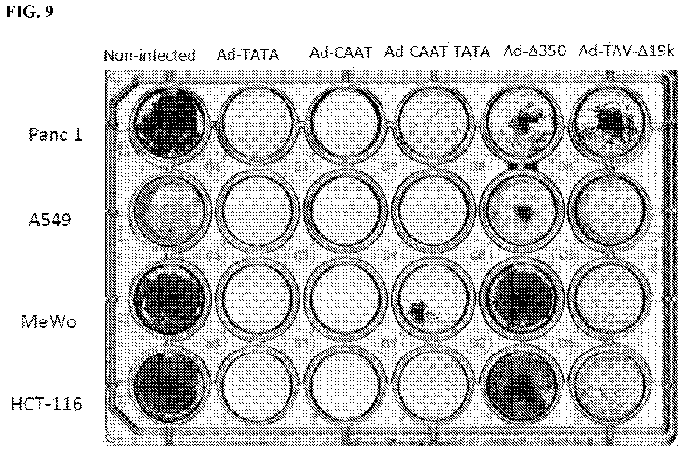

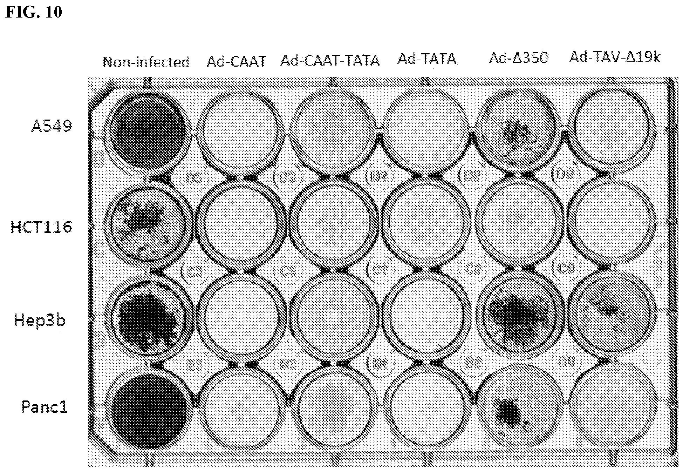

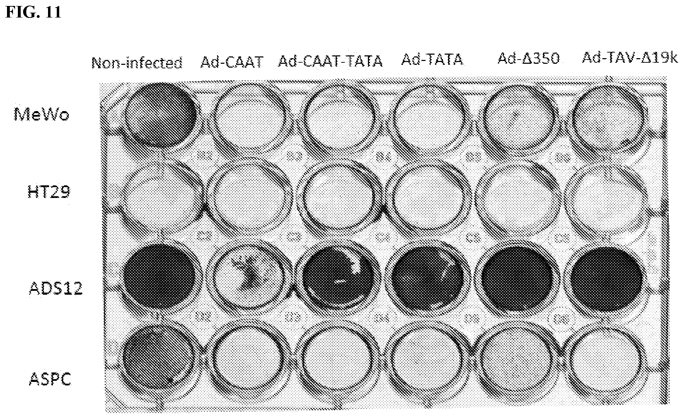

A panel of cancerous cell lines were infected with Ad-CAAT, Ad-TATA, Ad-CAAT-TATA, or Ad-CAAT-mTATA, prepared as described in Example 1. The panel included A549, Panel, HCT116, Hep3b, ADS-12m ASPC 1, HT-29, and MeWo cells. Cells were infected at an MOI of 5 and stained with crystal violet 3-4 days after infection. As a control, the cell lines were either cultured without infection or infected with the previously described oncolytic virus Ad-TAV-Δ19k. Results are shown in **FIGs. 5-11****.** All human cancerous cell lines showed extensive cell death after infection, particularly by five days after infection, while the mouse cell line ADS-12 showed variable cell death after infection with each of the viruses.

Non-cancerous MRC5 cells (human lung fibroblasts) and WI38 cells were infected with Ad-Δ350 or Ad-TAV prepared as described in Example 1. Cells were infected at the indicated MOI and stained with crystal violet ten days after infection. As depicted in **FIG. 4B****,** as opposed to the cancerous cells that were killed within 4-5 days post infection, the non-cancerous cells remained viable as late as 10 days after infection.

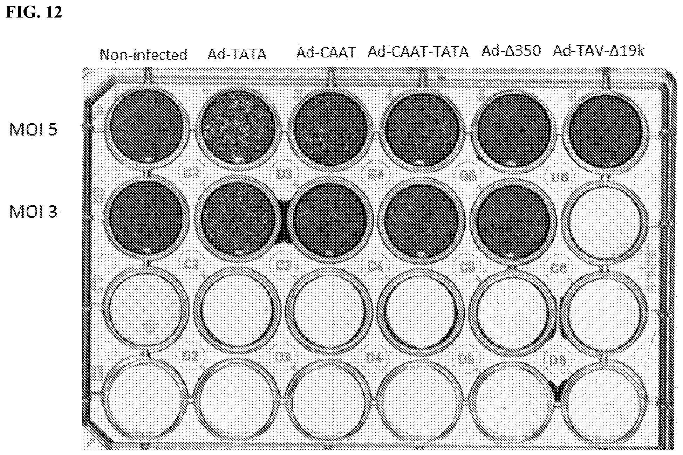

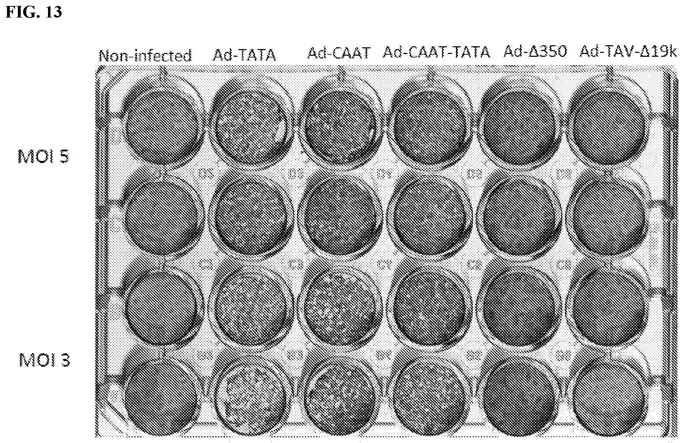

Non-cancerous WI38 cells were infected with Ad-CAAT, Ad-TATA, Ad-CAAT-TATA, Ad-Δ350, and Ad-TAV-Δ19k, prepared as described in Example 1, at 3 and 5 MOI and stained with crystal violet at four days **(****FIG. 12****)** or six days **(****FIG. 13****)** after infection. The results demonstrate that there was minimal cytotoxicity after infection for each virus.

Together, these results show that a 200 nucleotide region in Ad5, including the E1a TATA box, is required for Ad5-mediated cytotoxicity in non-cancerous cells, while this region is dispensable for Ad5-mediated cytotoxicity in tumor cells. Similarly, Ad5 viruses with deletions in the E1a promoter of either the TATA box alone, the CAAT box alone, or both the TATA and CAAT boxes showed cancer-selective cytotoxicity.

### Example 4: Therapeutic Transgene Expression From Ad-Δ350 In Normal And Cancerous Cells

Adenoviruses carrying the Δ350 deletion were further investigated for their potential to be armed with a therapeutic transgene in place of the viral E1b-19k gene. The following viruses were generated as described in Example 1: the virus Ad-Δ350-Δ19k, which carries the Δ350 deletion and has the 19k region deleted without the subsequent insertion of any transgene; the virus Ad-Δ350-mGM-CSF, which carries the Δ350 deletion and carries the gene for mouse GM-CSF cloned into the E1b-19k region between SalI and XhoI; and the virus Ad-TAV- Δ19k, which carries the TAV-255 deletion and has the 19k region deleted without the subsequent insertion of any transgene.

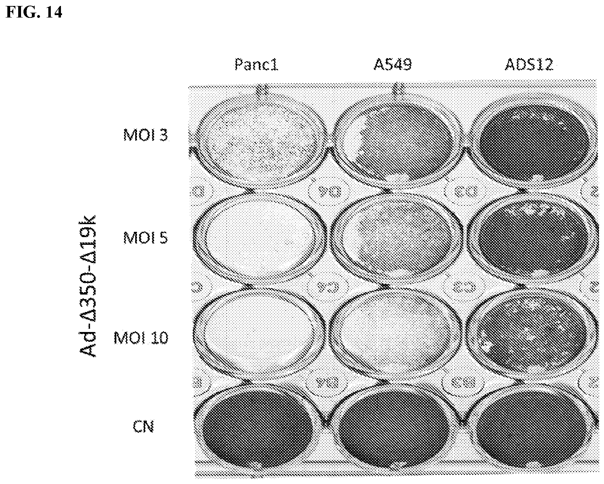

Cancerous Panc 1 cells, A549 cells, and ADS 12 cells (mouse lung carcinoma) were infected with Ad-Δ350-Δ19k at the indicated MOI and stained with crystal violet five days after infection. As depicted in **FIG. 14****,** the cancerous cell lines were killed in a dose-dependent manner.

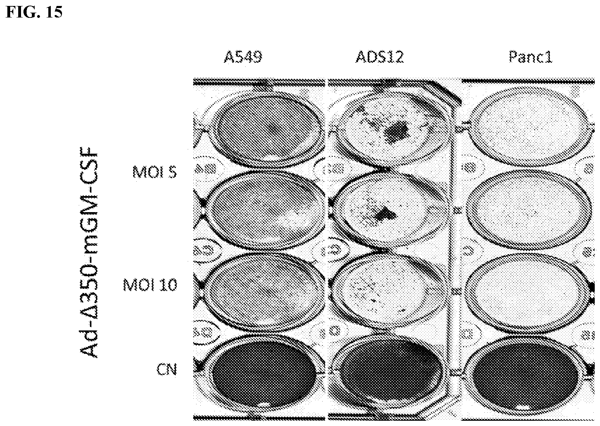

Cancerous Panc 1 cells, A549 cells, and ADS 12 cells were infected with Ad-Δ350-mGM-CSF at the indicated MOI and stained with crystal violet five days after infection. As shown in **FIG. 15**, the virus carrying the gene for mouse GM-CSF retained oncolytic activity.

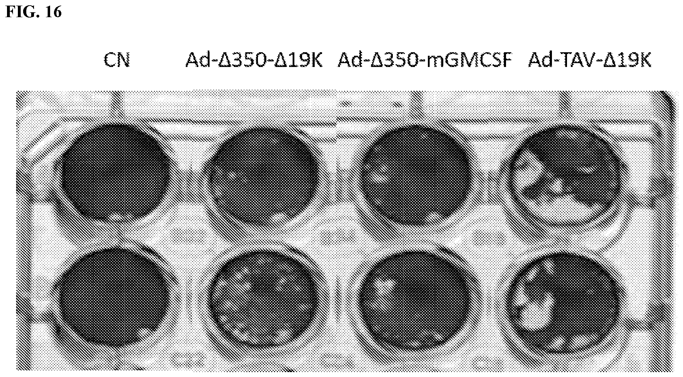

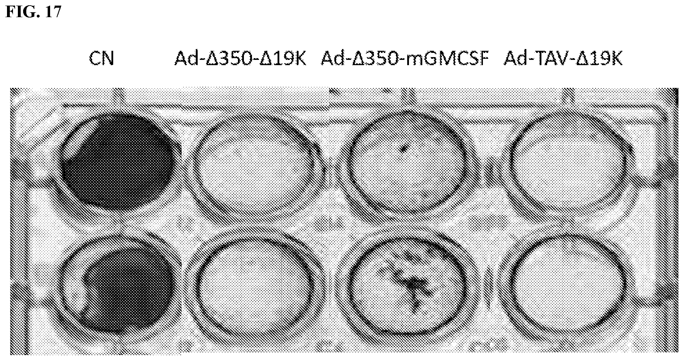

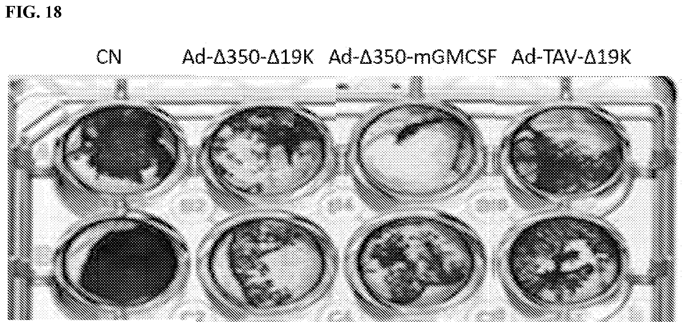

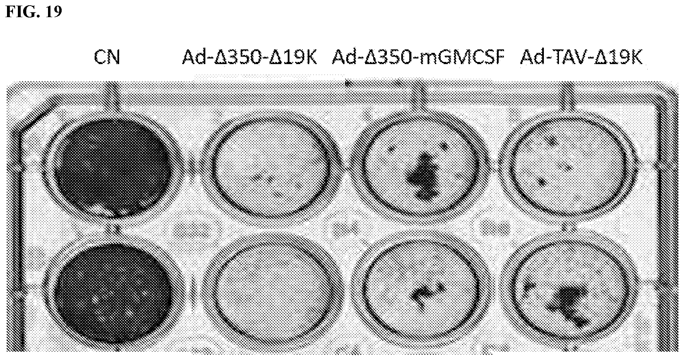

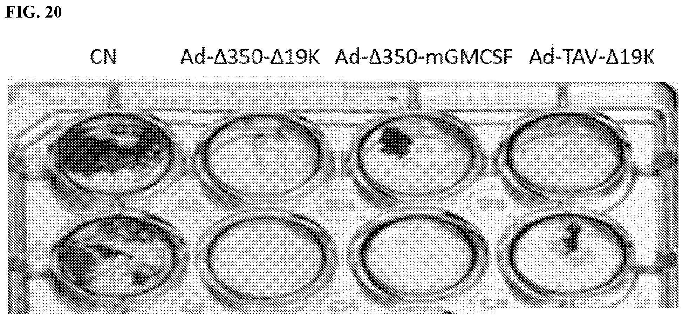

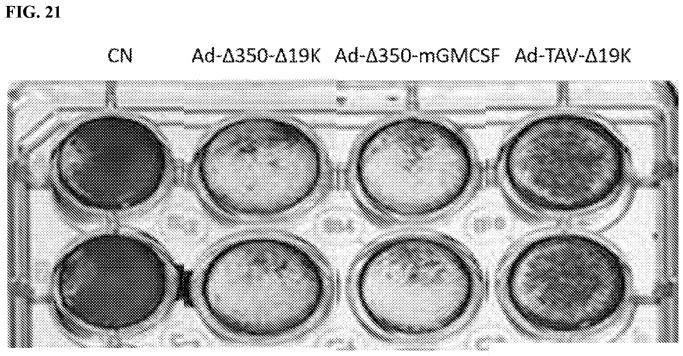

A549, HCT116, Hep3b, and MeWo cells were infected with Ad-Δ350-mGM-CSF, Ad-Δ350-Δ19k and Ad-TAV-Δ19k at an MOI of 5 and stained with crystal violet at 3 to 5 days after infection. As shown in **FIGs. 16-21****,** Ad-Δ350-mGM-CSF maintained cytolytic activity comparable to Ad-Δ350-Δ19k and Ad-TAV-Δ19k.

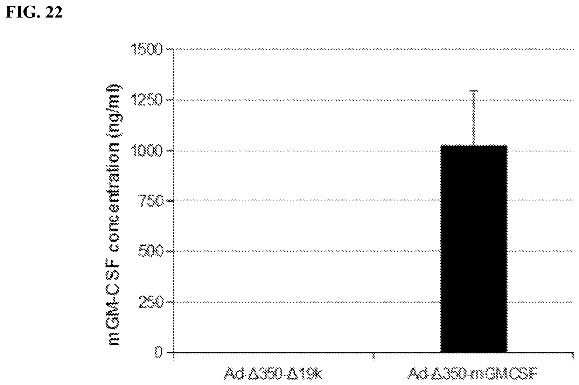

A549 cells were infected with Ad-Δ350-Δ19k, or Ad-Δ350-mGM-CSF viruses at 10 MOI. Conditioned media four days after infection was used in an ELISA for mGM-CSF. As shown in **FIG. 22**, Ad-Δ350-mGM-CSF induced expression of mGM-CSF.

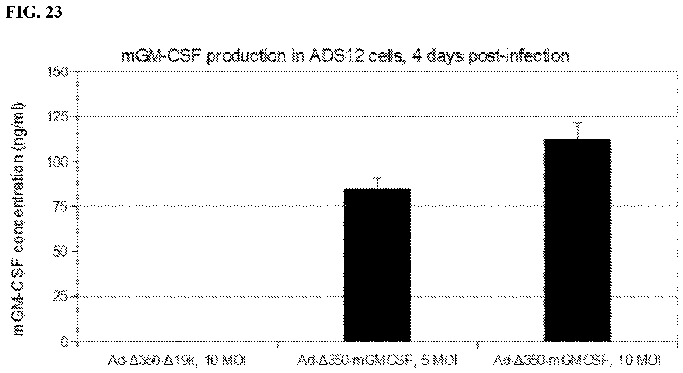

ADS12 cells were infected with Ad-Δ350-Δ19k or Ad-Δ350-mGM-CSF at the indicated MOI, and conditioned media four days after infection was used in an ELISA for mGM-CSF. As shown in **FIG. 23**, Ad-Δ350-mGM-CSF induced expression of mGM-CSF in this mouse cancer cell line.

Together, these results show that a 200 nucleotide region in Ad5, including the E1a TATA and CAAT boxes, is required for therapeutic transgene expression from an E1b-19k expression site in non-cancerous cells, while this region is dispensable for therapeutic transgene expression from an E1b-19k expression site in tumor cells.

### Example 5: Anti-Cancer Activity Of Ad-Δ350

This Example describes the anti-cancer activity of recombinant adenoviruses with TATA box and/or CAAT box deletions produced as described in Example 1.

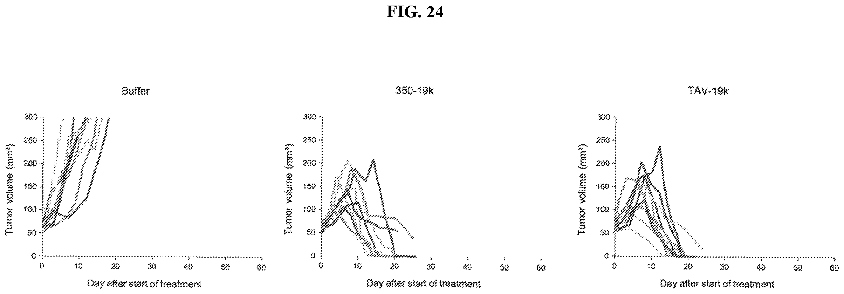

Mice (strain 129S4) were injected subcutaneously with ADS-12 cells (mouse lung cancer) and allowed to form tumors. After tumors reached a volume of approximately 50-100 mm³ the mice were randomized to treatment with Ad-Δ350-Δ19k, Ad-TAV-Δ19k (as a positive control for an effective oncolytic virus), or the buffer (as a negative control). The mice were dosed with intratumoral injections of the indicated treatment given every four days for three doses. As shown in **FIG. 24****,** mice treated with the buffer had rapid tumor growth while mice treated with either Ad-Δ350-Δ19k or Ad-TAV-Δ19k had reductions in their tumor size and in many cases no detectable remaining tumors.

This results suggest that Ad-Δ350-Δ19k, carrying a deletion that removes both the CAAT box and the TATA box of the promoter for the viral E1A gene, is effective cancer treatment.

### Example 6: TATA box deletion in Ad35

This Example describes the production of recombinant type 35 (Ad35) adenoviruses with deletions in the E1a promoter region that include a TATA box.

The E1a promoter of adenovirus type 35 (Ad35) contains a TATA box at nucleotides corresponding to nucleotides 477 to 484 of SEQ ID NO: 24. A recombinant Ad35 adenovirus was generated with the TATA box deleted by conversion of the natural sequence of 
TTTTACGTAGGTGTCAGCTGATCGCTAGGGTATTTATACCTCAGGGTTTGTGTCAAGAGGCCA
CTCTT (SEQ ID NO: 18; TATA box underlined)
 to
TTTTACGTAGGTGTCAGCTGATCGCTAGGGCCTCAGGGTTTGTGTCAAGAGGCCACTCTT (SEQ ID NO: 19).

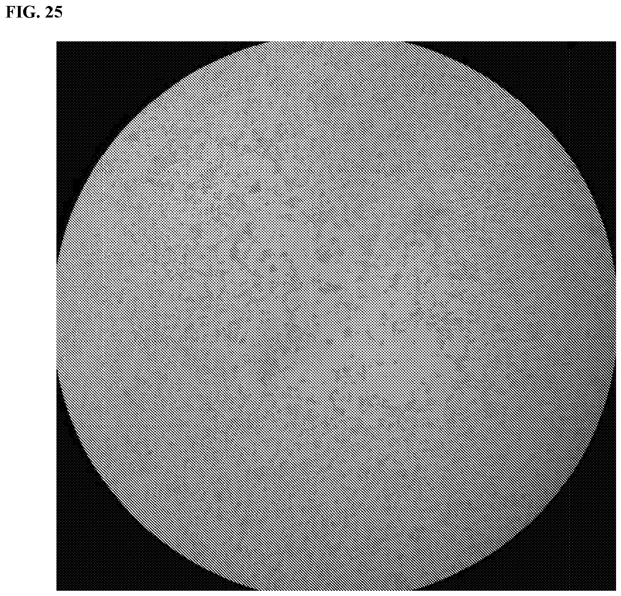

HEK-293 cells were transfected with genomes for the TATA-deleted Ad35 virus and, as show in **FIG. 25****,** developed a cytopathic effect indicative of viral growth. These results suggest that a recombinant Ad35 adenovirus was generated with the TATA box deleted may be suitable as an oncolytic virus.

## Claims

1. A recombinant oncolytic virus comprising:
(i) a modified TATA box-based promoter operably linked to a gene, wherein the modified TATA box-based promoter lacks a functional TATA box and permits selective expression of the gene in a cancer cell; and/or
(ii) a modified CAAT box-based promoter operably linked to a gene, wherein the modified CAAT box-based promoter lacks a functional CAAT box and permits selective expression of the gene in a cancer cell;
wherein the recombinant oncolytic virus is selected from a recombinant vaccinia virus, adenovirus, adeno-associated virus (AAV), herpes simplex virus 1 (HSV1), myxoma virus, reovirus, poliovirus, vesicular stomatitis virus (VSV), measles virus (MV), and Newcastle disease virus (NDV);
the modified TATA box-based promoter is an early gene promoter;
the modified CAAT box-based promoter is an early gene promoter;
wherein the gene is a therapeutic transgene and/or the early gene; and
the modification included in the modified TATA box-based promoter or CAAT box-based promoter does not comprise an addition, of or a substitution with, a separate, functional promoter sequence.

2. The recombinant oncolytic virus of claim 1, wherein the recombinant oncolytic virus is a recombinant oncolytic adenovirus,
optionally wherein the recombinant oncolytic virus is selected from a type 5 adenovirus and a type 35 adenovirus.

3. The recombinant oncolytic virus of claim 1 or 2, wherein:
i) the modified TATA box-based promoter is an E1a promoter, E1b promoter, or E4 promoter,
ii) the modification included in the modified TATA box-based promoter comprises a deletion of the entire TATA box,
iii) the modified CAAT box-based promoter is an E1a promoter, E1b promoter, or E4 promoter, or
iv) the modification included in the modified CAAT box-based promoter comprises a deletion of the entire CAAT box.

4. The recombinant oncolytic virus of any one of claims 1-3, wherein the virus comprises:
i) a deletion of nucleotides corresponding to -27 to -24 of an E1a promoter,
optionally wherein the virus comprises a deletion of nucleotides corresponding to -31 to -24 of the E1a promoter, in which case
optionally wherein the virus comprises a deletion of nucleotides corresponding to -44 to +54 of the E1a promoter, in which case
further optionally wherein the virus comprises a deletion of nucleotides corresponding to -146 to +54 of the E1a promoter;
ii) a deletion of nucleotides corresponding to 472 to 475 of an Ad5 genome (SEQ ID NO: 8),
optionally wherein the virus comprises a deletion of nucleotides corresponding to 468 to 475 of the Ad5 genome (SEQ ID NO: 8), in which case
optionally wherein the virus comprises a deletion of nucleotides corresponding to 455 to 552 of the Ad5 genome (SEQ ID NO: 8), in which case
further optionally wherein the virus comprises a deletion of nucleotides corresponding to 353 to 552 of the Ad5 genome (SEQ ID NO: 8);
iii) a polynucleotide deletion that results in a virus comprising the sequence CTAGGACTG (SEQ ID NO: 7), AGTGCCCG (SEQ ID NO: 12) and/or TATTCCCG (SEQ ID NO: 13);
iv) a deletion of nucleotides corresponding to -76 to -68 of an E1a promoter;
v) a deletion of nucleotides corresponding to 423 to 431 of an Ad5 genome (SEQ ID NO: 8);
vi) a polynucleotide deletion that results in a virus comprising the sequence TTCCGTGGCG (SEQ ID NO: 14); and/or
vii) a deletion of nucleotides corresponding to 477 to 484 of an Ad35 genome (SEQ ID NO: 24).

5. The recombinant oncolytic virus of any one of claims 1-4, further comprising a nucleotide sequence encoding the therapeutic transgene.

6. The recombinant oncolytic virus of any one of claims 1-5, wherein the recombinant oncolytic virus selectively:
i) replicates in the cancer cell, and/or has cytolytic activity in the cancer cell.

7. The recombinant oncolytic virus of any one of claims 2-6, wherein the recombinant oncolytic virus selectively expresses E1a and/or E1b in the cancer cell.

8. A pharmaceutical composition comprising the recombinant oncolytic virus of any one of claims 1-7 and at least one pharmaceutically acceptable carrier or diluent.

9. The recombinant oncolytic virus of any one of claims 1-7 for use in a method of treating cancer in a subject in need thereof, the method comprising administering to the subject an effective amount of the recombinant oncolytic virus to treat the cancer.

10. The recombinant oncolytic virus for use according to claim 9, wherein the cancer is selected from anal cancer, basal cell carcinoma, bladder cancer, bone cancer, brain cancer, breast cancer, carcinoma, cholangiocarcinoma, cervical cancer, colon cancer, colorectal cancer, endometrial cancer, gastroesophageal cancer, gastrointestinal (GI) cancer, gastrointestinal stromal tumor, hepatocellular carcinoma, gynecologic cancer, head and neck cancer, hematologic cancer, kidney cancer, leukemia, liver cancer, lung cancer, lymphoma, melanoma, merkel cell carcinoma, mesothelioma, neuroendocrine cancer, non-small cell lung cancer, ovarian cancer, pancreatic cancer, pediatric cancer, prostate cancer, renal cell carcinoma, sarcoma, skin cancer, small cell lung cancer, squamous cell carcinoma of the skin, stomach cancer, testicular cancer and thyroid cancer.

11. The recombinant oncolytic virus for use according to any one of claims 9-10, wherein the effective amount of the recombinant oncolytic virus is 10²-10¹⁵ plaque forming units (pfus), optionally wherein the subject is a human.

12. A method of engineering an oncolytic virus, the method comprising modifying a viral TATA box-based promoter operably linked to a gene such that the modified TATA box-based promoter lacks a functional TATA box and permits selective expression of the gene in a cancer cell and/or modifying a viral CAAT box-based promoter operably linked to a gene such that the modified CAAT box-based promoter lacks a functional CAAT box and permits selective expression of the gene in a cancer cell;
wherein the oncolytic virus is selected from a recombinant vaccinia virus, adenovirus, adeno-associated virus (AAV), herpes simplex virus 1 (HSV1), myxoma virus, reovirus, poliovirus, vesicular stomatitis virus (VSV), measles virus (MV), and Newcastle disease virus (NDV);
wherein the gene is a therapeutic transgene and/or the early gene;
the modified TATA box-based promoter is an early gene promoter;
the modified CAAT box-based promoter is an early gene promoter; and the modification included in the modified TATA box-based promoter or CAAT box-based promoter does not comprise an addition, of or a substitution with, a separate, functional promoter sequence.

13. An isolated nucleic acid comprising a nucleotide sequence selected from SEQ ID NO: 3, SEQ ID NO: 15, SEQ ID NO: 16, SEQ ID NO: 17, SEQ ID NO: 20, SEQ ID NO: 21, SEQ ID NO: 22 and SEQ ID NO: 23.

14. The isolated nucleic acid of claim 13, wherein the isolated nucleic acid comprises the nucleotide sequence of SEQ ID NO: 3, optionally wherein the isolated nucleic acid comprises the nucleotide sequence of SEQ ID NO: 4.

15. A host cell comprising the isolated nucleic acid of any one of claims 13-14.

16. A method of producing a recombinant virus comprising:
(a) growing the host cell of claim 15 under conditions to produce the recombinant virus; and
(b) purifying the recombinant virus.

## Patentansprüche

1. Rekombinantes onkolytisches Virus, umfassend:
(i) einen modifizierten, auf einer TATA-Box basierenden Promotor, der funktionell mit einem Gen verknüpft ist, wobei dem modifizierten, auf einer TATA-Box basierenden Promotor eine funktionelle TATA-Box fehlt und er die selektive Expression des Gens in einer Krebszelle ermöglicht; und/oder
(ii) einen modifizierten, auf einer CAAT-Box basierenden Promotor, der funktionell mit einem Gen verknüpft ist, wobei dem modifizierten, auf einer CAAT-Box basierenden Promotor eine funktionelle CAAT-Box fehlt und er die selektive Expression des Gens in einer Krebszelle ermöglicht;
wobei das rekombinante onkolytische Virus aus einem rekombinanten Vacciniavirus, Adenovirus, adenoassoziiertem Virus (AAV), Herpes-simplex-Virus 1 (HSV1), Myxomavirus, Reovirus, Poliovirus, Vesikuläre-Stomatitis-Virus (VSV), Masernvirus (MV) und Newcastle-Disease-Virus (NDV) ausgewählt ist;
der modifizierte, auf einer TATA-Box basierende Promotor ein Early-Gen-Promotor ist;
der modifizierte, auf einer CAAT-Box basierende Promotor ein Early-Gen-Promotor ist;
wobei das Gen ein therapeutisches Transgen und/oder das Early-Gen ist;
und die im modifizierten, auf einer TATA-Box basierenden Promotor oder auf einer CAAT-Box basierenden Promotor enthaltene Modifikation weder eine Addition einer separaten, funktionellen Promotorsequenz noch eine Substitution damit umfasst.

2. Rekombinantes onkolytisches Virus nach Anspruch 1, wobei das rekombinante onkolytische Virus ein rekombinantes onkolytisches Adenovirus ist,
wobei das rekombinante onkolytische Virus optional aus einem Adenovirus Typ 5 und einem Adenovirus Typ 35 ausgewählt ist.

3. Rekombinantes onkolytisches Virus nach Anspruch 1 oder 2, wobei:
i) der modifizierte, auf einer TATA-Box basierende Promotor ein E1a-Promotor, E1b-Promotor oder E4-Promotor ist,
ii) die im modifizierten, auf einer TATA-Box basierenden Promotor enthaltene Modifikation eine Deletion der gesamten TATA-Box umfasst,
iii) der modifizierte, auf einer CAAT-Box basierende Promotor ein E1a-Promotor, E1b-Promotor oder E4-Promotor ist, oder
iv) die im modifizierten, auf einer CAAT-Box basierenden Promotor enthaltene Modifikation eine Deletion der gesamten CAAT-Box umfasst.

4. Rekombinantes onkolytisches Virus nach einem der Ansprüche 1 bis 3, wobei das Virus umfasst:
i) eine Deletion von Nukleotiden, die -27 bis -24 eines Ela-Promotors entsprechen, wobei das Virus optional eine Deletion von Nukleotiden umfasst, die -31 bis -24 des E1a-Promotors entsprechen, wobei das Virus optional eine Deletion von Nukleotiden umfasst, die -44 bis +54 des E1a-Promotors entsprechen, wobei in diesem Fall
ferner optional wobei das Virus eine Deletion von Nukleotiden umfasst, die -146 bis +54 des E1a-Promotors entsprechen;
ii) eine Deletion von Nukleotiden, die 472 bis 475 eines Ad5-Genoms entsprechen (SEQ ID NO: 8),
optional wobei das Virus eine Deletion von Nukleotiden umfasst, die 468 bis 475 des Ad5-Genoms (SEQ ID NO: 8) entsprechen, wobei in diesem Fall optional das Virus eine Deletion von Nukleotiden umfasst,
die 455 bis 552 des Ad5-Genoms (SEQ ID NO: 8) entsprechen, wobei in diesem Fall ferner optional das Virus eine Deletion von Nukleotiden umfasst, die 353 bis 552 des Ad5-Genoms (SEQ ID NO: 8) entsprechen;
iii) eine Polynukleotiddeletion, die zu einem Virus führt, das die Sequenz CTAGGACTG (SEQ ID NO: 7), AGTGCCCG (SEQ ID NO: 12) und/oder TATTCCCG (SEQ ID NO: 13) umfasst;
iv) eine Deletion von Nukleotiden, die -76 bis -68 eines E1a-Promotors entsprechen;
v) eine Deletion von Nukleotiden, die 423 bis 431 eines Ad5-Genoms (SEQ ID NO: 8) entsprechen;
vi) eine Polynukleotiddeletion, die zu einem Virus führt, das die Sequenz TTCCGTGGCG (SEQ ID NO: 14) umfasst; und/oder
vii) eine Deletion von Nukleotiden, die 477 bis 484 eines Ad35-Genoms entsprechen (SEQ ID NO: 24) entsprechen.

5. Rekombinantes onkolytisches Virus nach einem der Ansprüche 1 bis 4, das ferner eine Nukleotidsequenz umfasst, die für das therapeutische Transgen codiert.

6. Rekombinantes onkolytisches Virus nach einem der Ansprüche 1 bis 5, wobei das rekombinante onkolytische Virus selektiv:
i) sich in der Krebszelle repliziert und/oder zytolytische Aktivität in der Krebszelle besitzt.

7. Rekombinantes onkolytisches Virus nach einem der Ansprüche 2 bis 6, wobei das rekombinante onkolytische Virus selektiv E1a und/oder E1b in der Krebszelle exprimiert.

8. Pharmazeutische Zusammensetzung, die das rekombinante onkolytische Virus nach einem der Ansprüche 1 bis 7 und mindestens einen pharmazeutisch verträglichen Träger oder ein pharmazeutisch verträgliches Verdünnungsmittel umfasst.

9. Rekombinantes onkolytisches Virus nach einem der Ansprüche 1 bis 7 zur Verwendung in einem Verfahren zum Behandeln von Krebs bei einem Individuum, das dies benötigt, wobei das Verfahren das Verabreichen einer wirksamen Menge des rekombinanten onkolytischen Virus an das Individuum zur Behandlung des Krebses umfasst.

10. Rekombinantes onkolytisches Virus zur Verwendung nach Anspruch 9, wobei der Krebs aus Analkrebs, Basalzellkarzinom, Blasenkrebs, Knochenkrebs, Hirnkrebs, Brustkrebs, Karzinom, Cholangiokarzinom, Gebärmutterhalskrebs, Dickdarmkrebs, kolorektalem Krebs, Gebärmutterschleimhautkrebs, gastroösophagealem Krebs, gastrointestinalem (GI) Krebs, gastrointestinalem Stromatumor, hepatozellulärem Karzinom, gynäkologischem Krebs, Kopf- und Halskrebs, hämatologischem Krebs, Nierenkrebs, Leukämie, Leberkrebs, Lungenkrebs, Lymphom, Melanom, Merkelzellkarzinom, Mesotheliom, neuroendokrinem Krebs, nicht kleinzelligem Lungenkrebs, Eierstockkrebs, Bauchspeicheldrüsenkrebs, Krebs bei Kindern, Prostatakrebs, Nierenzellkarzinom, Sarkom, Hautkrebs, kleinzelligem Lungenkrebs, Plattenepithelkarzinom der Haut, Magenkrebs, Hodenkrebs und Schilddrüsenkrebs ausgewählt ist.

11. Rekombinantes onkolytisches Virus zur Verwendung nach einem der Ansprüche 9 bis 10, wobei die wirksame Menge des rekombinanten onkolytischen Virus 10² bis 10¹⁵ plaquebildende Einheiten (PFU) beträgt, wobei es sich bei dem Individuum optional um einen Menschen handelt.

12. Verfahren zum Entwickeln eines onkolytischen Virus, wobei das Verfahren das Modifizieren eines viralen, auf einer TATA-Box basierenden Promotors, der funktionell mit einem Gen verknüpft ist, sodass dem modifizierten, auf einer TATA-Box basierenden Promotor eine funktionelle TATA-Box fehlt und er die selektive Expression des Gens in einer Krebszelle ermöglicht, und/oder das Modifizieren eines viralen, auf einer CAAT-Box basierenden Promotors, der funktionell mit einem Gen verknüpft ist, sodass dem modifizierten, auf einer CAAT-Box basierenden Promotor eine funktionelle CAAT-Box fehlt und er die selektive Expression des Gens in einer Krebszelle ermöglicht, umfasst;
wobei das onkolytische Virus aus einem rekombinanten Vacciniavirus, Adenovirus, adenoassoziiertem Virus (AAV), Herpes-simplex-Virus 1 (HSV1), Myxomavirus, Reovirus, Poliovirus, Vesikuläre-Stomatitis-Virus (VSV), Masernvirus (MV) und Newcastle-Disease-Virus (NDV) ausgewählt ist;
wobei das Gen ein therapeutisches Transgen und/oder das Early-Gen ist;
der modifizierte, auf einer TATA-Box basierende Promotor ein Early-Gen-Promotor ist;
der modifizierte, auf einer CAAT-Box basierende Promotor ein Early-Gen-Promotor ist; und die im modifizierten, auf einer TATA-Box basierenden Promotor oder auf einer CAAT-Box basierenden Promotor enthaltene Modifikation weder eine Addition einer separaten, funktionellen Promotorsequenz noch eine Substitution damit umfasst.

13. Isolierte Nukleinsäure, die eine Nukleotidsequenz umfasst, die aus SEQ ID NO: 3, SEQ ID NO: 15, SEQ ID NO: 16, SEQ ID NO: 17, SEQ ID NO: 20, SEQ ID NO: 21, SEQ ID NO: 22 und SEQ ID NO: 23 ausgewählt ist.

14. Isoliertes Nukleinsäuremolekül nach Anspruch 13, wobei das isolierte Nukleinsäuremolekül die Nukleotidsequenz von SEQ ID NO: 3 umfasst, optional wobei die isolierte Nukleinsäure die Nukleotidsequenz von SEQ ID NO: 4 umfasst.

15. Wirtszelle, die die Nukleinsäure nach einem der Ansprüche 13 bis 14 umfasst.

16. Verfahren zum Herstellen eines rekombinanten Virus, umfassend:
(a) Züchten der Wirtszelle nach Anspruch 15 unter Bedingungen zur Herstellung des rekombinanten Virus; und
(b) Reinigen des rekombinanten Virus.

## Revendications

1. Virus oncolytique recombinant comprenant :
(i) un promoteur modifié basé sur la boîte TATA lié de manière opérationnelle à un gène, dans lequel le promoteur modifié basé sur la boîte TATA est dépourvu d'une boîte TATA fonctionnelle et permet l'expression sélective du gène dans une cellule cancéreuse ; et/ou
(ii) un promoteur modifié basé sur la boîte CAAT lié de manière opérationnelle à un gène, dans lequel le promoteur modifié basé sur la boîte CAAT est dépourvu d'une boîte CAAT fonctionnelle et permet l'expression sélective du gène dans une cellule cancéreuse ;
dans lequel le virus oncolytique recombinant est choisi parmi un virus de la vaccine recombinant, un adénovirus, un virus adéno-associé (AAV), un virus de l'herpès simplex 1 (HSV1), un virus du myxome, un réovirus, un poliovirus, un virus de la stomatite vésiculaire (VSV), un virus de la rougeole (MV), et un virus de la maladie de Newcastle (NDV) ;
le promoteur modifié basé sur la boîte TATA est un promoteur de gène précoce ;
le promoteur modifié basé sur la boîte CAAT est un promoteur de gène précoce ;
dans lequel le gène est un transgène thérapeutique et/ou le gène précoce ; et la modification incluse dans le promoteur modifié basé sur la boîte TATA ou le promoteur modifié basé sur la boîte CAAT ne comprend pas l'ajout ou la substitution d'une séquence de promoteur fonctionnelle distincte.

2. Virus oncolytique recombinant selon la revendication 1, dans lequel le virus oncolytique recombinant est un adénovirus oncolytique recombinant,
éventuellement, dans lequel le virus oncolytique recombinant est choisi parmi un adénovirus de type 5 et un adénovirus de type 35.

3. Virus oncolytique recombinant selon la revendication 1 ou 2, dans lequel :
i) le promoteur modifié basé sur la boîte TATA est un promoteur Ela, un promoteur Elb ou un promoteur E4,
ii) la modification incluse dans le promoteur modifié basé sur la boîte TATA comprend une suppression de l'ensemble de la boîte TATA,
iii) le promoteur modifié basé sur la boîte CAAT est un promoteur Ela, un promoteur Elb ou un promoteur E4, ou
iv) la modification incluse dans le promoteur modifié basé sur la boîte CAAT comprend une suppression de l'ensemble de la boîte CAAT.

4. Virus oncolytique recombinant selon l'une quelconque des revendications 1 à 3, dans lequel le virus comprend :
i) une délétion de nucléotides correspondant à -27 à -24 d'un promoteur Ela, éventuellement dans lequel le virus comprend une délétion de nucléotides correspondant à -31 à -24 du promoteur Ela, auquel cas éventuellement dans lequel le virus comprend une délétion de nucléotides correspondant à -44 à +54 du promoteur Ela, auquel cas
dans lequel le virus comprend éventuellement en outre une délétion de nucléotides correspondant à -146 à +54 du promoteur Ela ;
ii) une délétion de nucléotides correspondant à 472 à 475 d'un génome Ad5 (SEQ ID NO: 8) ;
dans lequel le virus comprend éventuellement une délétion de nucléotides correspondant à 468 à 475 du génome Ad5 (SEQ ID NO: 8), auquel cas le virus comprend éventuellement une délétion de nucléotides
correspondant à 455 à 552 du génome Ad5 (SEQ ID NO: 8), auquel cas dans lequel le virus comprend éventuellement en outre une délétion de nucléotides correspondant à 353 à 552 du génome Ad5 (SEQ ID NO: 8) ;
iii) une délétion polynucléotidique qui aboutit à un virus comprenant la séquence CTAGGACTG (SEQ ID NO: 7), AGTGCCCG (SEQ ID NO: 12) et/ou TATTCCCG (SEQ ID NO: 13) ;
iv) une délétion de nucléotides correspondant à -76 à -68 d'un promoteur Ela ;
v) une délétion de nucléotides correspondant à 423 à 431 d'un génome Ad5 (SEQ ID NO: 8) ;
vi) une délétion polynucléotidique qui aboutit à un virus comprenant la séquence TTCCGTGGCG (SEQ ID NO: 14) ; et/ou
vii) une délétion de nucléotides correspondant à 477 à 484 d'un génome Ad35 (SEQ ID NO: 24).

5. Virus oncolytique recombinant selon l'une quelconque des revendications 1 à 4, comprenant en outre une séquence nucléotidique codant pour le transgène thérapeutique.

6. Virus oncolytique recombinant selon l'une quelconque des revendications 1 à 5, dans lequel le virus oncolytique recombinant sélectivement :
1) se réplique dans la cellule cancéreuse et/ou a une activité cytolytique dans la cellule cancéreuse.

7. Virus oncolytique recombinant selon l'une quelconque des revendications 2 à 6, dans lequel le virus oncolytique recombinant exprime sélectivement Ela et/ou Elb dans la cellule cancéreuse.

8. Composition pharmaceutique comprenant le virus oncolytique recombinant selon l'une quelconque des revendications 1 à 7 et au moins un transporteur ou un diluant pharmaceutiquement acceptable.

9. Virus oncolytique recombinant selon l'une quelconque des revendications 1 à 7 pour une utilisation dans un procédé de traitement du cancer chez un sujet qui en a besoin, le procédé comprenant l'administration au sujet d'une quantité efficace du virus oncolytique recombinant pour traiter le cancer.

10. Virus oncolytique recombinant destiné à être utilisé selon la revendication 9, dans lequel le cancer est choisi parmi le cancer anal, le carcinome basocellulaire, le cancer de la vessie, le cancer des os, le cancer du cerveau, le cancer du sein, le carcinome, le cholangiocarcinome, le cancer du col de l'utérus, le cancer du côlon, le cancer colorectal, le cancer de l'endomètre, le cancer gastro-oesophagien, le cancer gastro-intestinal (GI), la tumeur stromale gastro-intestinale, le carcinome hépatocellulaire, le cancer gynécologique, le cancer de la tête et du cou, le cancer hématologique, cancer de la tête et du cou, cancer hématologique, cancer du rein, leucémie, cancer du foie, cancer du poumon, lymphome, mélanome, carcinome à cellules de Merkel, mésothéliome, cancer neuroendocrinien, cancer du poumon non à petites cellules, cancer de l'ovaire, cancer du pancréas, cancer pédiatrique, cancer de la prostate, carcinome à cellules rénales, sarcome, cancer de la peau, cancer du poumon à petites cellules, carcinome épidermoïde de la peau, cancer de l'estomac, cancer du testicule et cancer de la thyroïde.

11. Virus oncolytique recombinant destiné à être utilisé selon l'une quelconque des revendications 9 à 10, dans lequel la quantité efficace du virus oncolytique recombinant est de 10² à 10¹⁵ unités formatrices de plaques (UFP), éventuellement dans lequel le sujet est un humain.

12. Procédé d'ingénierie d'un virus oncolytique, le procédé comprenant la modification d'un promoteur viral basé sur la boîte TATA lié de manière opérationnelle à un gène, de sorte que le promoteur modifié basé sur la boîte TATA est dépourvu d'une boîte TATA fonctionnelle et permet l'expression sélective du gène dans une cellule cancéreuse et/ou la modification d'un promoteur viral basé sur la boîte CAAT lié de manière opérationnelle à un gène, de sorte que le promoteur modifié basé sur la boîte CAAT est dépourvu d'une boîte CAAT fonctionnelle et permet l'expression sélective du gène dans une cellule cancéreuse ;
dans lequel le virus oncolytique est choisi parmi un virus de la vaccine recombinant, un adénovirus, un virus adéno-associé (AAV), un virus de l'herpès simplex 1 (HSV1), un virus du myxome, un réovirus, un poliovirus, un virus de la stomatite vésiculaire (VSV), un virus de la rougeole (MV), et un virus de la maladie de Newcastle (NDV) ;
dans lequel le gène est un transgène thérapeutique et/ou le gène précoce ;
le promoteur modifié basé sur la boîte TATA est un promoteur de gène précoce ;
le promoteur modifié basé sur la boîte CAAT est un promoteur de gène précoce ; et la modification incluse dans le promoteur modifié basé sur la boîte TATA ou le promoteur modifié basé sur la boîte CAAT ne comprend pas un ajout ou une substitution d'une séquence de promoteur fonctionnelle distincte.

13. Acide nucléique isolé comprenant une séquence de nucléotides choisie parmi les séquences SEQ ID NO: 3, SEQ ID NO: 15, SEQ ID NO: 16, SEQ ID NO: 17, SEQ ID NO: 20, SEQ ID NO: 21, SEQ ID NO: 22 et SEQ ID NO: 23.

14. Acide nucléique isolé selon la revendication 13, dans lequel l'acide nucléique isolé comprend la séquence SEQ ID N: 3, éventuellement dans lequel l'acide nucléique isolé comprend la séquence nucléotidique de SEQ ID NO: 4.

15. Cellule hôte comprenant l'acide nucléique isolé selon l'une quelconque des revendications 13 à 14.

16. Procédé de production d'un virus recombinant comprenant :
(a) la culture de la cellule hôte selon la revendication 15 dans des conditions permettant de produire le virus recombinant ; et
(b) la purification du virus recombinant.
